# EUROPEAN PATENT SPECIFICATION

(11) **EP 1 487 998 B1**
(45) Date of publication and mention of the grant of the patent: **06.06.2007**
(21) Application number: 03714858.2
(22) Date of filing: 19.03.2003
(51) Int. Cl.: C12Q 1/68

(54) **METHODS FOR DIAGNOSING AND TREATING SCHIZOPHRENIA**
VERFAHREN ZUR DIAGNOSE UND THERAPIE VON SCHIZOPHRENIE
METHODE POUR DIAGNOSTIQUER ET TRAITER LA SCHIZOPHRENIE

(30) Priority: 20.03.2002 US 366001 P
(43) Date of publication of application: 22.12.2004
(73) Proprietor: Novartis AG, 4056 Basel (CH); Novartis Pharma GmbH, 1230 Wien (AT)
(72) Inventor: BUXTON, Francis, Paul, Morristown, NJ 07960 (US); CARPENTER, William, Twitty, Columbia, MD 21044 (US); ROBERTS, Rosalinda, Cusido, Columbia, MD 21045 (US); TAMMINGA, Carol, Ann, Dallas, TX 75209 (US)
(74) Representative: Crawley, Patrick Edward
(86) International application number: PCT/EP2003/002875
(87) International publication number: WO 2003/078658

(56) References cited:
- WO-A-01/64834
- WO-A-01/64835
- US-B1- 6 197 069
- ZOROGLU S SALIH ET AL: "The possible pathophysiological role of plasma nitric oxide and adrenomedullin in schizophrenia" JOURNAL OF PSYCHIATRIC RESEARCH, vol. 36, no. 5, September 2002 (2002-09), pages 309-315, XP002262396 ISSN: 0022-3956
- SHAN JING ET AL: "Distribution of preproadrenomedullin mRNA in the rat central nervous system and its modulation by physiological stressors" JOURNAL OF COMPARATIVE NEUROLOGY, vol. 432, no. 1, 26 March 2001 (2001-03-26), pages 88-100, XP009021499 ISSN: 0021-9967
- DATABASE EMBL [Online] human DEPP, 1 May 1999 (1999-05-01) WATANABE: retrieved from EBI, accession no. np_008952 Database accession no. nm_007021 XP002262398
- TAKAHASHI KAZUHIRO: "Adrenomedullin from a pheochromocytoma to the eye: Implications of the adrenomedullin research for endocrinology in the 21st century" TOHOKU JOURNAL OF EXPERIMENTAL MEDICINE, vol. 193, no. 2, February 2001 (2001-02), pages 79-114, XP009021566 ISSN: 0040-8727 cited in the application
- KHAN S ET AL: "Effects of acute restraint stress on endogenous adrenomedullin levels" NEUROREPORT, vol. 10, no. 13, 9 September 1999 (1999-09-09), pages 2829-2833, XP009021493 ISSN: 0959-4965
- COLES LEEANNE S ET AL: "Cold shock domain proteins repress transcription from the GM-CSF promoter" NUCLEIC ACIDS RESEARCH, vol. 24, no. 12, 1996, pages 2311-2317, XP002262397 ISSN: 0305-1048

### SEQUENCE LISTING

<110> Buxton, Francis Paul
   Carpenter, William Twitty
   Roberts, Rosalina Cusido
   Tamminga, carol Ann
<120> Method for Diagnosing and Treating Schizophrenia
<130> DC4-32423A
<140> 10/507,858
   <141> 2004-09-15
<150> PCT/EP03/02875
   <151> 2003-03-19
<150> 60/366,001
   <151> 2003-03-20
<160> 82
<170> FastSEQ for windows version 4.0
<210> 1
   <211> 52
   <212> PRT
   <213> Homo Sapiens
<400> 1
   Tyr Arg Gln Ser Met Asn Asn Phe Gln Gly Leu Arg Ser Phe Gly Cys
    1               5                  10                  15
   Arg Phe Gly Thr Cys Thr Val Gln Lys Leu Ala His Gln Ile Tyr Gln
               20                  25                  30
   Phe Thr Asp Lys Asp Lys Asp Asn Val Ala Pro Arg Ser Lys Ile Ser
           35                  40                  45
   Pro Gln Gly Tyr
       50
<210> 2
   <211> 1449
   <212> DNA
   <213> Homo Sapiens
<220>
   <223> Synthesized Probes
<400> 2
   ctggatagaa cagctcaagc cttgccactt cgggcttctc actgcagctg ggcttggact 60
   tcggagtttt gccattgcca gtgggacgtc tgagactttc tccttcaagt acttggcaga 120
   tcactctctt agcagggtct gcgcttcgca gccgggatga agctggtttc cgtcgccctg 180
   atgtacctgg gttcgctcgc cttcctaggc gctgacaccg ctcggttgga tgtcgcgtcg 240
   gagtttcgaa agaagtggaa taagtgggct ctgagtcgtg ggaagaggga actgcggatg 300
   tccagcagct accccaccgg gctcgctgac gtgaaggccg ggcctgccca gacccttatt 360
   cggccccagg acatgaaggg tgcctctcga agccccgaag acagcagtcc ggatgccgcc 420
   cgcatccgag tcaagcgcta ccgccagagc atgaacaact tccagggcct ccggagcttt 480
   ggctgccgct tcgggacgtg cacggtgcag aagctggcac accagatcta ccagttcaca 540
   gataaggaca aggacaacgt cgcccccagg agcaagatca gcccccaggg ctacggccgc 600
   cggcgccggc gctccctgcc cgaggccggc ccgggtcgga ctctggtgtc ttctaagcca 660
   caagcacacg gggctccagc ccccccgagt ggaagtgctc cccactttct ttaggattta 720
   ggcgcccatg gtacaaggaa tagtcgcgca agcatcccgc tggtgcctcc cgggacgaag 780
   gacttcccga gcggtgtggg gaccgggctc tgacagccct gcggagaccc tgagtccggg 840
   aggcaccgtc cggcggcgag ctctggcttt gcaagggccc ctccttctgg gggcttcgct 900
   tccttagcct tgctcaggtg caagtgcccc agggggcggg gtgcagaaga atccgagtgt 960
   ttgccaggct taaggagagg agaaactgag aaatgaatgc tgagaccccc ggagcagggg 1020
   tctgagccac agccgtgctc gcccacaaac tgatttctca cggcgtgtca ccccaccagg 1080
   gcgcaagcct cactattact tgaactttcc aaaacctaaa gaggaaaagt gcaatgcgtg 1140
   ttgtacatac agaggtaact atcaatattt aagtttgttg ctgtcaagat tttttttgta 1200
   acttcaaata tagagatatt tttgtacgtt atatattgta ttaagggcat tttaaaagca 1260
   attatattgt cctcccctat tttaagacgt gaatgtctca gcgaggtgta aagttgttcg 1320
   ccgcgtggaa tgtgagtgtg tttgtgtgca tgaaagagaa agactgatta cctcctgtgt 1380
   ggaagaagga aacaccgagt ctctgtataa tctatttaca taaaatgggt gatatgcgaa 1440
   cagcaaacc                                                         1449
<210> 3
   <211> 212
   <212> PRT
   <213> Homo Sapiens
<400> 3
   Met Arg Ser Arg Leu Leu Leu Ser Val Ala His Leu Pro Thr Ile Arg
   1 5 10 15
   Glu Thr Thr Glu Glu Met Leu Leu Gly Gly Pro Gly Gln Glu Pro Pro
   20 25 30
   Pro Ser Pro Ser Leu Asp Asp Tyr Val Arg Ser Ile Ser Arg Leu Ala
   35 40 45
   Gln Pro Thr Ser Val Leu Asp Lys Ala Thr Ala Gln Gly Gln Pro Arg
   50 55 60
   Pro Pro His Arg Pro Ala Gln Ala Cys Arg Lys Gly Arg Pro Ala Val
   65 70 75 80
   Ser Leu Arg Asp Ile Thr Ala Arg Phe Ser Gly Gln Gln Pro Thr Leu
   85 90 95
   Pro Met Ala Asp Thr Val Asp Pro Leu Asp Trp Leu Phe Gly Glu Ser
   100 105 110
   Gln Glu Lys Gln Pro Ser Gln Arg Asp Leu Pro Arg Arg Thr Gly Pro
   115 120 125
   Ser Ala Gly Leu Trp Gly Pro His Arg Gln Met Asp Ser Ser Lys Pro
   130 135 140
   Met Gly Ala Pro Arg Gly Arg Leu Cys Glu Ala Arg Met Pro Gly His
   145 150 155 160
   Ser Leu Ala Arg Pro Pro Gln Asp Gly Gln Gln Ser Ser Asp Leu Arg
   165 170 175
   Ser Trp Thr Phe Gly Gln Ser Ala Gln Ala Met Ala Ser Arg His Arg
   180 185 190
   Pro Arg Pro Ser Ser Val Leu Arg Thr Leu Tyr Ser His Leu Pro Val
   195 200 205
   Ile His Glu Leu
   210
<210> 4
   <211> 2114
   <212> DNA
   <213> Homo sapiens
<400> 4
   cacactgctc agggaagagc ctgctacggt ggactgtgag actcagtgca ctgtcctcct 60
   cccagcgacc ccacgctgga ccccctgccg gaccctccac ccttcggccc ccaagcttcc 120
   caggggcttc ctttggactg gactgtccct gctcatccat tctcctgcca cccccagacc 180
   tcctcagctc caggttgcca cctcctctcg ccagagtgat gaggtcccgg cttctgctct 240
   ccgtggccca tctgcccaca attcgggaga ccacggagga gatgctgctt gggggtcctg 300
   gacaggagcc cccaccctct cctagcctgg atgactacgt gaggtctata tctcgactgg 360
   cacagcccac ctctgtgctg gacaaggcca cggcccaggg ccaacccagg ccaccccaca 420
   ggccagccca ggcctgccgg aagggccgcc ctgctgtgtc cctgcgagac atcaccgcac 480
   gtttcagtgg ccagcagccc acactgccca tggctgatac tgtggacccc ctggactggc 540
   tttttgggga gtcccaggaa aagcagccaa gccagaggga cctgccaagg aggactggcc 600
   cctctgctgg cctctggggt ccacatagac agatggacag cagcaagccc atgggggccc 660
   ccagagggag gctctgtgaa gccaggatgc ctgggcattc cctggcaaga ccaccgcagg 720
   atgggcagca gagctctgac ctaagaagct ggacttttgg gcagtctgcc caagccatgg 780
   cctcccgcca ccgcccccgc cccagcagtg tcctcagaac actctactcg cacctcccgg 840
   tgatccatga actctgaccc ctccccagta aaggcttctg tagagagcat gctgggtctg 900
   catctcctct cgtctcctcc atggtggtca ctgcccctgg caggtctctg aaagggaaat 960
   gcttttctgc agaggcccct tcttgggcag ttcacagtta gacccacccc ctctgaatat 1020
   gataacagcc tgtttcacat gaggagatgt taccaatccc gttcgctctg acccttgctg 1080
   gctgatcacc ttgagcaact tacttaacat ctgtgttcct cagtttctca tgggtaatat 1140
   agggataatt actggcacct gcctcccagg ccattctgac gtgtaccgca tataggagcc 1200
   cactggctga gtagctacca tcatcgctgg tggggaaact ggtggtaggg gtgtgagggt 1260
   agtgggggtg tcagccccca ggtgtttcag aacaaggcct cgggcactcc caagtctgcc 1320
   tcttggctcc caccctcaaa gcccatgttc tgtgaggccc aagagaacac atggagtctt 1380
   agcaaatgca ctaatgtatt ccgggggact gtcacctggc accactgggg cactctgctg 1440
   gctacaactc atacgtcctg tggtggcatt gggagagttc ccccatgatg agggccaaga 1500
   tagaatctgt accactcagt gctaccatcc ccacccctac accacttcca cacaggggcc 1560
   tcatggcatg gtcagggtcc cagctgtggg tgagagcagg gcactgtcca gctgtccact 1620
   ggggaagtca agatgtccta aggcccaggc cagggcatct ggagtctgaa ggaccctagt 1680
   tcctagaggc atctggcagc aagaaggtga ggcatcaggg aacgggaatc aggctgggac 1740
   tgatcagagg tgaagggaca gagagaggag aggaggaaga ttgagctggg gcaacagcca 1800
   agctcacctg gcaggtctct gccacctcct tctctgtgag ctgtcagtct aggttattct 1860
   ctttttttgt ggctattttt aattgctttg gatttgttaa atgttttctg tcttctgtta 1920
   agtgtgtttt ctctggagat agaatgtaaa ccatattaaa aggaaaaagt ttcagacaag 1980
   caattaccca gtttccttat ctataaaatg gggacatcag caatgttctt cacaccctgc 2040
   aagggctggg aattgcgcat gtgaacttgg agctgcattt atgagcactg tagacaaatg 2100
   tttgtatctg tcac                                                   2114
<210> 5
   <211> 372
   <212> PRT
   <213> Homo Sapiens
<400> 5
   Met Ser Glu Ala Gly Glu Ala Thr Thr Thr Thr Thr Thr Thr Leu Pro
   1               5                   10                  15
   Gln Ala Pro Thr Glu Ala Ala Ala Ala Ala Pro Gln Asp Pro Ala Pro
               20                  25                  30
   Lys Ser Pro Val Gly Ser Gly Ala Pro Gln Ala Ala Ala Pro Ala Pro
           35                  40                  45
   Ala Ala His Val Ala Gly Asn Pro Gly Gly Asp Ala Ala Pro Ala Ala
       50                  55                  60
   Thr Gly Thr Ala Ala Ala Ala Ser Leu Ala Thr Ala Ala Gly Ser Glu
   65                  70                  75                  80
   Asp Ala Glu Lys Lys Val Leu Ala Thr Lys Val Leu Gly Thr Val Lys
                   85                  90                  95
   Trp Phe Asn Val Arg Asn Gly Tyr Gly Phe Ile Asn Arg Asn Asp Thr
               100                 105                 110
   Lys Glu Asp Val Phe Val His Gln Thr Ala Ile Lys Lys Asn Asn Pro
           115                 120                 125
   Arg Lys Tyr Leu Arg Ser Val Gly Asp Gly Glu Thr Val Glu Phe Asp
       130                 135                 140
   Val Val Glu Gly Glu Lys Gly Ala Glu Ala Ala Asn Val Thr Gly Pro
   145                 150                 155                 160
   Asp Gly Val Pro Val Glu Gly Ser Arg Tyr Ala Ala Asp Arg Arg Arg
                   165                 170                 175
   Tyr Arg Arg Gly Tyr Tyr Gly Arg Arg Arg Gly Pro Pro Arg Asn Tyr
               180                 185                 190
   Ala Gly Glu Glu Glu Glu Glu Gly Ser Gly Ser Ser Glu Gly Phe Asp
           195                 200                 205
   Pro Pro Ala Thr Asp Arg Gln Phe Ser Gly Ala Arg Asn Gln Leu Arg
       210                 215                 220
   Arg Pro Gln Tyr Arg Pro Gln Tyr Arg Gln Arg Arg Phe Pro Pro Tyr
   225                 230                 235                 240
   His Val Gly Gln Thr Phe Asp Arg Arg Ser Arg Val Leu Pro His Pro
                   245                 250                 255
   Asn Arg Ile Gln Ala Gly Glu Ile Gly Glu Met Lys Asp Gly Val Pro
               260                 265                 270
   Glu Gly Ala Gln Leu Gln Gly Pro Val His Arg Asn Pro Thr Tyr Arg
           275                 280                 285
   Pro Arg Tyr Arg Ser Arg Gly Pro Pro Arg Pro Arg Pro Ala Pro Ala
       290                 295                 300
   Val Gly Glu Ala Glu Asp Lys Glu Asn Gln Gln Ala Thr Ser Gly Pro
   305                 310                 315                 320
   Asn Gln Pro Ser Val Arg Arg Gly Tyr Arg Arg Pro Tyr Asn Tyr Arg
                   325                 330                 335
   Arg Arg Pro Arg Pro Pro Asn Ala Pro Ser Gln Asp Gly Lys Glu Ala
               340                 345                 350
   Lys Ala Gly Glu Ala Pro Thr Glu Asn Pro Ala Pro Pro Thr Gln Gln
           355                 360                 365
   Ser Ser Ala Glu
       370
<210> 6
   <211> 1392
   <212> DNA
   <213> Homo sapiens
<400> 6
   gaattccggc acgaagctcg agccgcctcc gccgcgcgac cccacctcgg ccgccgccgc 60
   ctgcgccgcg agatccgccc cggcctcccc gagagcgagc cccggccgcc gcgaccacca 120
   gccgcgctaa ccgccgacca accgccaccg aggcgcctga gcgagagcag aggaggagga 180
   ggcatgagtg aggcgggcga ggccaccacc accaccacca ccaccctccc gcaggctccg 240
   acggaggcgg ccgccgcggc tccccaggac cccgcgccca agagcccggt gggcagcggt 300
   gcgccccagg ccgcggcccc ggcgcccgcc gcccacgtcg caggaaaccc cggtggggac 360
   gcggcccccg cagccacggg caccgcggcc gccgcctctt tagccaccgc cgccggcagc 420
   gaagacgcgg agaaaaaagt tctcgccacc aaagtccttg gcactgtcaa atggttcaac 480
   gtcagaaatg gatatggatt tataaatcga aatgacacca aagaagatgt atttgtacat 540
   cagactgcca tcaagaagaa taacccacgg aaatatctgc gcagtgtagg agatggagaa 600
   actgtagagt ttgatgtggt tgaaggagag aagggtgcag aagctgccaa tgtgactggc 660
   ccggatggag ttcctgtgga agggagtcgt tacgctgcag atcggcgccg ttacagacgt 720
   ggctactatg gaaggcgccg tggccctccc cggaattacg ctggggagga ggaggaggaa 780
   gggagcggca gcagtgaagg atttgacccc cctgccactg ataggcagtt ctctggggcc 840
   cggaatcagc tgcgccgccc ccagtatcgc cctcagtacc ggcagcggcg gttcccgcct 900
   taccacgtgg gacagacctt tgaccgtcgc tcacgggtct taccccatcc caacagaata 960
   caggctggtg agattggaga gatgaaggat ggagtcccag agggagcaca acttcaggga 1020
   ccggttcatc gaaatccaac ttaccgccca aggtaccgta gcaggggacc tcctcgccca 1080
   cgacctgccc cagcagttgg agaggctgaa gataaagaaa atcagcaagc caccagtggt 1140
   ccaaaccagc cgtctgttcg ccgtggatac cggcgtccct acaattaccg gcgtcgcccg 1200
   cgtcctccta acgctccttc acaagatggc aaagaggcca aggcaggtga agcaccaact 1260
   gagaaccctg ctccacccac ccagcagagc agtgctgagt aacaccaggc tcctcaggca 1320
   ccttcaccat cggcaggtga cctaaagaat taatgaccat tcagaaataa agcaaaaagc 1380
   aggccggaat tc                                                     1392
<210> 7
   <211> 23
   <212> DNA
   <213> unknown
<220>
   <223> synthesized Probes
<400> 7
   cagggctgct tttaactctg gta 23
<210> 8
   <211> 24
   <212> DNA
   <213> unknown
<220>
   <223> synthesized Probes
<400> 8
   gggtggaatc atattggaac atgt 24
<210> 9
   <211> 27
   <212> DNA
   <213> unknown
<220>
   <223> Synthesized Probes
<400> 9
   ttttaatctc tcgactgaat ggacttt 27
<210> 10
   <211> 24
   <212> DNA
   <213> unknown
<220>
   <223> Synthesized Probes
<400> 10
   cacaactcat cccctcttgt gtac 24
<210> 11
   <211> 24
   <212> DNA
   <213> unknown
<220>
   <223> synthesized Probes
<400> 11
   tgtgttttca gcaaattcca gatt 24
<210> 12
   <211> 32
   <212> DNA
   <213> unknown
<220>
   <223> Synthesized Probes
<400> 12
   tgtcatcata aaaatagaaa gtaggaaaga tt 32
<210> 13
   <211> 25
   <212> DNA
   <213> Unknown
<220>
   <223> Synthesized Probes
<400> 13
   ttctgttctc aaatgctaaa tgcaa 25
<210> 14
   <211> 24
   <212> DNA
   <213> unknown
<220>
   <223> Synthesized Probes
<400> 14
   gggaatgttg atgtcattca gaaa 24
<210> 15
   <211> 24
   <212> DNA
   <213> Unknown
<220>
   <223> Synthesized Probes
<400> 15
   gcctgttgca gagtttttct gtaa 24
<210> 16
   <211> 29
   <212> DNA
   <213> Unknown
<220>
   <223> synthesized Probes
<400> 16
   ttccaatatt ctctaacacg tacactgaa 29
<210> 17
   <211> 28
   <212> DNA
   <213> unknown
<220>
   <223> synthesized Probes
<400> 17
   ggtagaagaa acaatgcaag acatacat 28
<210> 18
   <211> 25
   <212> DNA
   <213> unknown
<220>
   <223> synthesized Probes
<400> 18
   cccttgttaa tgatgcctgt tctat 25
<210> 19
   <211> 27
   <212> DNA
   <213> unknown
<220>
   <223> synthesized Probes
<400> 19
   ggaaatgtac ctgaaaagga ttttaga 27
<210> 20
   <211> 22
   <212> DNA
   <213> unknown
<220>
   <223> synthesized Probes
<400> 20
   actacatccc ctccatgtgc at 22
<210> 21
   <211> 26
   <212> DNA
   <213> unknown
<220>
   <223> Synthesized Probes
<400> 21
   agtgcagatt tatactcctg acgtgt 26
<210> 22
   <211> 36
   <212> DNA
   <213> unknown
<220>
   <223> Synthesized Probes
<400> 22
   caagacttat aatcatgaaa tacagaatta aaagtt 36
<210> 23
   <211> 20
   <212> DNA
   <213> unknown
<220>
   <223> synthesized Probes
<400> 23
   tgtccgtccc tgtttttgct 20
<210> 24
   <211> 21
   <212> DNA
   <213> unknown
<220>
   <223> synthesized Probes
<400> 24
   caccctgcct ttccctagag a 21
<210> 25
   <211> 22
   <212> DNA
   <213> unknown
<220>
   <223> synthesized Probes
<400> 25
   ggaagatctc agtgcagagg ct 22
<210> 26
   <211> 21
   <212> DNA
   <213> unknown
<220>
   <223> synthesized Probes
<400> 26
   gatctccttg gccacaatgg t 21
<210> 27
   <211> 23
   <212> DNA
   <213> unknown
<220>
   <223> synthesized Probes
<400> 27
   tggatggagg acagattgtg act 23
<210> 28
   <211> 21
   <212> DNA
   <213> unknown
<220>
   <223> Synthesized Probes
<400> 28
   aatgaggagc atggtgacca g 21
<210> 29
   <211> 21
   <212> DNA
   <213> unknown
<220>
   <223> Synthesized Probes
<400> 29
   tcgcccacaa actgatttct c 21
<210> 30
   <211> 22
   <212> DNA
   <213> unknown
<220>
   <223> Synthesized Probes
<400> 30
   acgcattgca cttttcctct tt 22
<210> 31
   <211> 20
   <212> DNA
   <213> unknown
<220>
   <223> synthesized Probes
<400> 31
   ccctcctgct gtgctcttct 20
<210> 32
   <211> 24
   <212> DNA
   <213> unknown
<220>
   <223> Synthesized Probes
<400> 32
   gtgacagaca cacatcagcc acta 24
<210> 33
   <211> 34
   <212> DNA
   <213> unknown
<220>
   <223> Synthesized Probes
<400> 33
   gatagaatta actcgtattt ttctatggtt ttaa 34
<210> 34
   <211> 21
   <212> DNA
   <213> unknown
<220>
   <223> Synthesized Probes
<400> 34
   ggagaaagcc aagggaaaca a 21
<210> 35
   <211> 26
   <212> DNA
   <213> unknown
<220>
   <223> Synthesized Probes
<400> 35
   tccttacttg tcatcagaga cgaact 26
<210> 36
   <211> 25
   <212> DNA
   <213> unknown
<220>
   <223> Synthesized Probes
<400> 36
   aaaaatctgt agggaatgca tcctt 25
<210> 37
   <211> 25
   <212> DNA
   <213> unknown
<220>
   <223> Synthesized Probes
<400> 37
   aaaggaagga aactcctgac agtct 25
<210> 38
   <211> 31
   <212> DNA
   <213> unknown
<220>
   <223> Synthesized Probes
<400> 38
   gcttcagaga tacaaataca gtgtaatacc a 31
<210> 39
   <211> 24
   <212> DNA
   <213> unknown
<220>
   <223> synthesized Probes
<400> 39
   ctggagtaga gttcctggtt gctt 24
<210> 40
   <211> 25
   <212> DNA
   <213> unknown
<220>
   <223> Synthesized Probes
<400> 40
   aaagacactt caacctcaaa accaa 25
<210> 41
   <211> 23
   <212> DNA
   <213> unknown
<220>
   <223> Synthesized Probes
<400> 41
   cctcagtggg ttcgttaaaa tca 23
<210> 42
   <211> 24
   <212> DNA
   <213> unknown
<220>
   <223> synthesized Probes
<400> 42
   ggcatttaat tgacctcaca gaga 24
<210> 43
   <211> 20
   <212> DNA
   <213> unknown
<220>
   <223> Synthesized Probes
<400> 43
   gtgccaatat gccctccaaa 20
<210> 44
   <211> 23
   <212> DNA
   <213> unknown
<220>
   <223> synthesized Probes
<400> 44
   ggcatctctt cagtgcaatt tct 23
<210> 45
   <211> 22
   <212> DNA
   <213> unknown
<220>
   <223> Synthesized Probes
<400> 45
   ttctgaaatg tgacccccaa gt 22
<210> 46
   <211> 28
   <212> DNA
   <213> unknown
<220>
   <223> Synthesized Probes
<400> 46
   gctaaatgca actgttcctt ttctataa 28
<210> 47
   <211> 21
   <212> DNA
   <213> unknown
<220>
   <223> synthesized Probes
<400> 47
   ggcaacattt ccgaagacct t 21
<210> 48
   <211> 25
   <212> DNA
   <213> unknown
<220>
   <223> Synthesized Probes
<400> 48
   gcatactcta aaacgcacag tgtga 25
<210> 49
   <211> 20
   <212> DNA
   <213> unknown
<220>
   <223> Synthesized Probes
<400> 49
   cctccctcca gtgtccacat 20
<210> 50
   <211> 22
   <212> DNA
   <213> unknown
<220>
   <223> Synthesized Probes
<400> 50
   aagcaggatt ctggacatgg aa 22
<210> 51
   <211> 30
   <212> DNA
   <213> unknown
<220>
   <223> Synthesized Probes
<400> 51
   aagtgctaat aattaactca accaggtcta 30
<210> 52
   <211> 28
   <212> DNA
   <213> unknown
<220>
   <223> Synthesized Probes
<400> 52
   ctaggtctat atccgtatga aatgcatt 28
<210> 53
   <211> 25
   <212> DNA
   <213> unknown
<220>
   <223> Synthesized Probes
<400> 53
   aagtcacaat gagtttgggc atatt 25
<210> 54
   <211> 27
   <212> DNA
   <213> unknown
<220>
   <223> synthesized Probes
<400> 54
   accatttcca gcctaaacta cataaaa 27
<210> 55
   <211> 26
   <212> DNA
   <213> unknown
<220>
   <223> Synthesized Probes
<400> 55
   cagctttgac tttatctcct gctctt 26
<210> 56
   <211> 20
   <212> DNA
   <213> unknown
<220>
   <223> Synthesized Probes
<400> 56
   ccctggaggc tgtgatctca 20
<210> 57
   <211> 26
   <212> DNA
   <213> unknown
<220>
   <223> Synthesized Probes
<400> 57
   gagtctgaag gaccctagtt cctaga 26
<210> 58
   <211> 22
   <212> DNA
   <213> Unknown
<220>
   <223> Synthesized Probes
<400> 58
   tctgtccctt cacctctgat ca 22
<210> 59
   <211> 23
   <212> DNA
   <213> unknown
<220>
   <223> Synthesized Probes
<400> 59
   aattgttttt cgtccgtttg gta 23
<210> 60
   <211> 23
   <212> DNA
   <213> unknown
<220>
   <223> Synthesized Probes
<400> 60 23
   aattgccata tacggccagt taa 23
<210> 61
   <211> 20
   <212> DNA
   <213> Unknown
<220>
   <223> Synthesized Probes
<400> 61 20
   gcaggagcct cactgtgcat 20
<210> 62
   <211> 20
   <212> DNA
   <213> unknown
<220>
   <223> synthesized Probes
<400> 62
   acagatgtgg ccccgttgta 20
<210> 63
   <211> 25
   <212> DNA
   <213> unknown
<220>
   <223> synthesized Probes
<400> 63
   tgtacacata tgaacgcaca acatg 25
<210> 64
   <211> 22
   <212> DNA
   <213> Unknown
<220>
   <223> synthesized Probes
<400> 64
   gacctcagga aaaggcttca ga 22
<210> 65
   <211> 20
   <212> DNA
   <213> unknown
<220>
   <223> Synthesized Probes
<400> 65
   gtccaaacca gccgtctgtt 20
<210> 66
   <211> 21
   <212> DNA
   <213> unknown
<220>
   <223> Synthesized Probes
<400> 66
   gcctctttgc catcttgtga a 21
<210> 67
   <211> 24
   <212> DNA
   <213> Unknown
<220>
   <223> Synthesized Probes
<400> 67
   aaagcctctg attgttgttt cctt 24
<210> 68
   <211> 20
   <212> DNA
   <213> Unknown
<220>
   <223> Synthesized Probes
<400> 68
   acgcccgaaa caccaaataa 20
<210> 69
   <211> 24
   <212> DNA
   <213> unknown
<220>
   <223> Synthesized Probes
<400> 69
   agcaacagga aactcatgga gatt 24
<210> 70
   <211> 25
   <212> DNA
   <213> unknown
<220>
   <223> Synthesized Probes
<400> 70
   gagcagcgct atggtacaga atact 25
<210> 71
   <211> 20
   <212> DNA
   <213> unknown
<220>
   <223> synthesized Probes
<400> 71
   cagcctcaaa acgggtcagt 20
<210> 72
   <211> 20
   <212> DNA
   <213> unknown
<220>
   <223> Synthesized Probes
<400> 72
   cttctttctc cgtccctccg 20
<210> 73
   <211> 22
   <212> DNA
   <213> unknown
<220>
   <223> Synthesized Probes
<400> 73
   aatatcccct cccagtcacc tt 22
<210> 74
   <211> 20
   <212> DNA
   <213> unknown
<220>
   <223> Synthesized Probes
<400> 74
   aaagctgctc ccatagccct 20
<210> 75
   <211> 22
   <212> DNA
   <213> unknown
<220>
   <223> Synthesized Probes
<400> 75
   tgacacctgt gcattagatg ca 22
<210> 76
   <211> 20
   <212> DNA
   <213> Unknown
<220>
   <223> Synthesized Probes
<400> 76
   catggtgtga gtctgggcct 20
<210> 77
   <211> 25
   <212> DNA
   <213> Unknown
<220>
   <223> Synthesized Probes
<400> 77
   tggtacaggg tgcctatttt agtca 25
<210> 78
   <211> 25
   <212> DNA
   <213> unknown
<220>
   <223> synthesized Probes
<400> 78
   tgtagtttcc aaaatgaacc cttgt 25
<210> 79
   <211> 24
   <212> DNA
   <213> unknown
<220>
   <223> Synthesized Probes
<400> 79
   aactgccttg tgttctgtga gaaa 24
<210> 80
   <211> 23
   <212> DNA
   <213> unknown
<220>
   <223> Synthesized Probes
<400> 80
   gcagcattgt aagttgtgat gca 23
<210> 81
   <211> 22
   <212> DNA
   <213> unknown
<220>
   <223> Synthesized Probes
<400> 81
   ttgaacactc actccacaac cc 22
<210> 82
   <211> 30
   <212> DNA
   <213> unknown
<220>
   <223> Synthesized Probes
<400> 82
   tttcacatca acaaacaaaa ttcattataa 30

## Description

### BACKGROUND

### 1. Field of the Invention

The present disclosure relates to genes correlated to schizophrenia and methods of using genes for diagnosis and treatment of schizophrenia.

### 2. Description of Related Art

Schizophrenia is a severe psychiatric disorder usually characterized by withdrawal from reality, illogical patterns of thinking, delusions and hallucinations, and accompanied in varying degrees by other emotional, behavioral, or intellectual disturbances. See Diagnostic and Statistical Manual of Mental Disorders, Fourth Edition, American Psychiatric Association, 273-315 (1994) (DSM-IV^{™}). However, as stated therein, no single symptom is pathognomonic of schizophrenia; the diagnosis involves recognition of a constellation of signs and symptoms associated with impaired occupational or social functioning. Id. Some detectable physiological changes have been reported, e.g., neuropathological and imaging studies depicting anatomical alterations associated with the disease. Arnold et al., Acta Neuropathol. (Berl) 92, 217- 231 (1996); Harrison, Brain 122, 593-624 (1999). Certain cellular aberrations have been observed and biochemical and RNA analyses have demonstrated alterations in some neurotransmitter pathways and presynaptic components. Id.; Benes, Brain Res. Rev. 31, 251-269 (2000).

At beginning stages and even at more advanced stages, schizophrenia can involve subtle behavioral changes and subtle and/or undetectable changes at the cellular and/or molecular levels in nervous system structure and function. This lack of detectable neurological defect distinguishes schizophrenia from other well-defined neurological disorders in which anatomical or biochemical pathologies are clearly manifest. Thus, there is a need for non-subjective modalities for screening and diagnosis of schizophrenia. Moreover, identification of the causative defects and the neuropathologies of schizophrenia are needed in order to enable clinicians to evaluate and prescribe appropriate courses of treatment to cure or ameliorate the symptoms of schizophrenia at early stages or when symptoms are obscured. Indeed, there are few effective therapies for the disease and its molecular basis is still not well understood.

Methods have been designed to survey alterations in mRNA expression in order to search for genes disregulated in various diseases and disorders. In organisms for which the complete genome is known, it is possible to analyze the transcripts of all genes within the cell. With other organisms, such as human, for which there is an increasing knowledge of the genome, it is possible to simultaneously monitor large numbers of genes within a cell. DNA microarray analysis is a technique that permits the quantitative measurement of the transcriptional expression of several thousand genes simultaneously. This technique permits one to generate profiles of gene expression patterns in both patients suffering from schizophrenia and control individuals. Accordingly, determination of abnormal levels of gene expression provides a signpost for therapeutic intervention.

Techniques for modifying RNA levels and activities involve ribozymes, antisense species, and RNA aptamers and small molecule promoter modulators. Ribozymes are RNAs capable of catalyzing RNA cleavage reactions, and some can be designed to specifically cleave a particular target mRNA. Ribozyme methods include exposing a cell to, inducing expression in a cell, etc. of such RNA ribozyme molecules. Activity of a target RNA (preferably mRNA) species, specifically its rate of translation, can be inhibited by the application of antisense nucleic acids. "Antisense" nucleic acids are nucleic acids capable of hybridizing to a sequence specific portion of the target RNA, e.g., its translation initiation region by virtue of some sequence that is complementary to a coding and/or non-coding region. The antisense nucleic acid can be oligonucleotides that are double-stranded or single-stranded, RNA or DNA or a modification or derivative thereof, which can be produced intracellularly by transcription of exogenous, introduced sequences in controllable quantities sufficient to perturb translation of the target RNA.

The above described techniques are emerging as an effective means for reducing the expression of specific gene products and may therefore prove to be uniquely useful in a number of therapeutic, diagnostic and research applications for the modulation of genes that are disregulated in schizophrenic patients.

### SUMMARY

In one aspect, a method for screening for schizophrenia in a population is provided which includes determining, in members of the population, the magnitude of expression of a gene selected from the group consisting of the gene encoding decidual protein induced by progesterone (DEPP), the gene encoding adrenomedullin and the gene encoding cold shock domain protein A (csdA) in a sample and comparing the magnitude of expression to a baseline magnitude of expression of the gene, wherein increased gene expression indicates the presence of schizophrenia. The sample may be taken from the brain, spinal cord, lymphatic fluid, blood, urine or feces. In a preferred embodiment the sample is taken from the anterior cingulated. In another preferred embodiment, the population is human. In another aspect, a method for diagnosing schizophrenia in a host is provided which includes determining the magnitude of expression of a gene selected from the group consisting of the gene encoding decidual protein induced by progesterone (DEPP), the gene encoding adrenomedullin and the gene encoding cold shock domain protein A (csdA) in a sample and comparing the magnitude of expression to a baseline magnitude of expression of the gene, wherein increased gene expression indicates the presence of schizophrenia.

A method for treating schizophrenia in a host is disclosed which includes lowering expression of a gene selected from the group consisting of the gene encoding decidual protein induced by progesterone (DEPP), the gene encoding adrenomedullin and the gene encoding cold shock domain protein A (csdA) by administering to the host an expression lowering amount of antisense oligonucleotide or an expression lowering amount small-inhibitory RNA (siRNA). In an embodiment of this method, the host is human.

Also disclosed is a method for treating schizophrenia in a host which includes lowering expression of a gene selected from the group consisting of the gene encoding decidual protein induced by progesterone (DEPP), the gene encoding adrenomedullin and the gene encoding cold shock domain protein A (csdA) by administering to the host an expression lowering amount of a ribozyme which cleaves RNA associated with expression of the gene. In another aspect, a method for treating schizophrenia in a host is disclosed which includes lowering expression of a gene selected from the group consisting of the gene encoding decidual protein induced by progesterone (DEPP), the gene encoding adrenomedullin and the gene encoding cold shock domain protein A (csdA) by administering one or more nucleic acid molecules designed to promote triple helix formation with said gene. In another aspect, a method for treating schizophrenia is disclosed which includes reducing the amount of available DEPP, adrenomedullin and/or csdA in a patient by administering an effective amount of anti-DEPP, anti-adrenomedullin and/or anti-csdA antibody. In an embodiment of the above methods, the host is a human.

In another aspect, a method of screening for compounds which are useful in the treatment of schizophrenia is provided which includes operatively linking a reporter gene which expresses a detectable protein to a regulatory sequence for a gene selected from the group consisting of DEPP, adrenomedullin and csdA to produce a reporter construct, transfecting a cell with the reporter construct, exposing the transfected cell to a test compound, and comparing the level of expression of the reporter gene after exposure to the test compound to the level of expression before exposure to the test compound, wherein a lower level of expression after exposure is indicative of a compound useful for the treatment of schizophrenia.

In another aspect, the use of a transgenic nonhuman animal is provided which stably includes in its genome an increased copy number of a gene selected from the group consisting of the gene encoding decidual protein induced by progesterone (DEPP), the gene encoding adrenomedullin and the gene encoding cold shock domain protein A (csdA) wherein the gene is expressed at higher than baseline levels and the animal exhibits abnormal behavior. In one aspect, the transgenic animal includes in its genome a gene selected from the group consisting of the gene encoding decidual protein induced by progesterone (DEPP), the gene encoding adrenomedullin and the gene encoding cold shock domain protein A (csdA) wherein expression of the gene is enhanced by at least one alteration in regulatory sequences of the gene such that the gene is expressed at higher than baseline levels and the animal exhibits abnormal behavior. In a preferred embodiment, the one or more alterations comprises substitution of a promoter having a higher rate of expression than the native promoter of the gene, in a more preferred embodiment the promoter is an inducible promoter. In another aspect, the transgenic nonhuman knockout animal includes a homozygous disruption in one or more genes selected from the group consisting of the gene encoding decidual protein induced by progesterone (DEPP), the gene encoding adrenomedullin and the gene encoding cold shock domain protein A (csdA), wherein said homozygous disruption prevents the expression of the gene, and wherein said homozygous disruption results in the transgenic knockout animal exhibiting decreased expression levels of the one or more genes as compared to a wild-type animal. In one aspect, the above transgenic nonhuman animals are utilized to screen for therapeutic agents that modulate symptoms of schizophrenia by administering a candidate compound to the transgenic nonhuman animals and determining the effect of the compound on symptoms associated with schizophrenia. In a preferred embodiment of the above aspects, the transgenic nonhuman animal is a mammal.

### BRIEF DESCRIPTION OF THE FIGURES

FIG. 1 depicts a nucleic acid sequence encoding preproadrenomedullin.
FIG. 2 depicts a nucleic acid sequence encoding DEPP.
FIG. 3 depicts a nucleic acid sequence encoding cold shock domain protein A (csdA).

### DETAILED DESCRIPTION OF PREFERRED EMBODIMENTS

The present disclosure is based on the surprising discovery that three genes, namely, the gene encoding decidual protein induced by progesterone (DEPP), the gene encoding adrenomedullin and the gene encoding cold shock domain protein A (csdA) are associated with schizophrenia in affected individuals. More particularly, these three genes are upregulated in the anterior cingulate of schizophrenic patients as compared to normal patients. Accordingly, methods for the diagnosis, screening and evaluation of schizophrenia are provided in accordance with the present invention. For example, assays for determination of increased levels of expression of the DEPP, csdA and adrenomedullin genes are provided. Moreover, nucleic acid molecules encoding DEPP, csdA and adrenomedullin can be used as diagnostic hybridization probes or used to design primers for diagnostic PCR analysis for the identification of DEPP, csdA or adrenomedullin gene mutations, allelic variations and regulatory defects in the DEPP, csdA or adrenomedullin genes. As used herein, "diagnosis" is intended to generally apply to individuals while "screening" is generally applicable to populations or individuals. Both terms also encompass in vitro methods using for instance isolated tissue or isolated cells or cells grown in culture. The invention also encompasses antibodies to the DEPP, csdA and adrenomedullin gene products that can be used to decrease available plasma levels of these proteins, as well as nucleotide sequences that can be used to inhibit gene expression (e.g., antisense, siRNA and ribozyme molecules), and gene or regulatory sequence binding or replacement constructs designed to reduce or enhance gene expression (e.g., triple helix forming moieties or expression constructs that place the genes under the control of a strong promoter system).

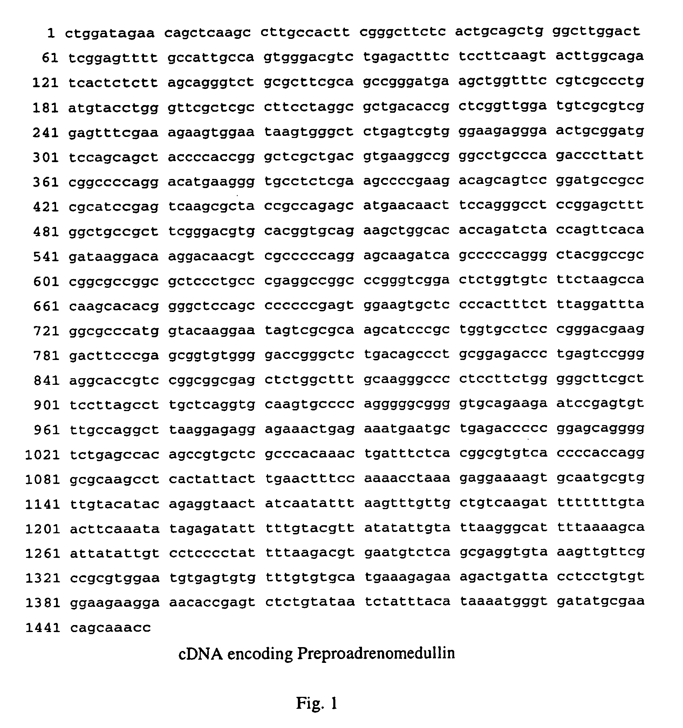

Adrenomedullin is a potent vasodilator peptide consisting of 52 amino acids with the following sequence: YRQSMNNFQG LRSFGCRFGT CTVQKLAHQI YQFTDKDKDN VAPRSKISPQ GY (Seq. Id. No.1). The precursor, called preproadrenomedullin, is 185 amino acids long. It was originally discovered from a human pheochromocytoma, and belongs to the calcitonin gene-related peptide (CGRP) family. cDNA encoding the preproadrenomedullin protein is depicted in Fig. 1 (Seq. Id. No. 2). Adrenomedullin is produced and secreted by various types of cells, for example, vascular endothelial and smooth muscle cells, cardiomyocytes, fibroblasts, macrophages, neurons, glial cells, and retinal pigment epithelial cells. Expression of adrenomedullin is induced by hypoxia and proinflammatory cytokines. In addition to vasodilator actions, this peptide has central inhibitory actions on water drinking and salt appetite, effects on the secretion of some hormones and cytokines, inotropic actions and effects on cell growth and apoptosis. Adrenomedullin is also produced by various non-endocrine tumors, as well as endocrine tumors, and acts as a growth stimulatory factor for the tumor cells. Adrenomedullin seems to be involved in the pathophysiology of many diseases, including ischemic heart diseases, inflammatory diseases, tumors, and even eye diseases. Takahashi, Tohoku J. Exp. Med., 193 (2) 79-114 (2001). As used herein, the "gene encoding adrenomedulin" or "adrenomedulin gene" is meant to encompass preproadrenomedullin and adrenomedullin.

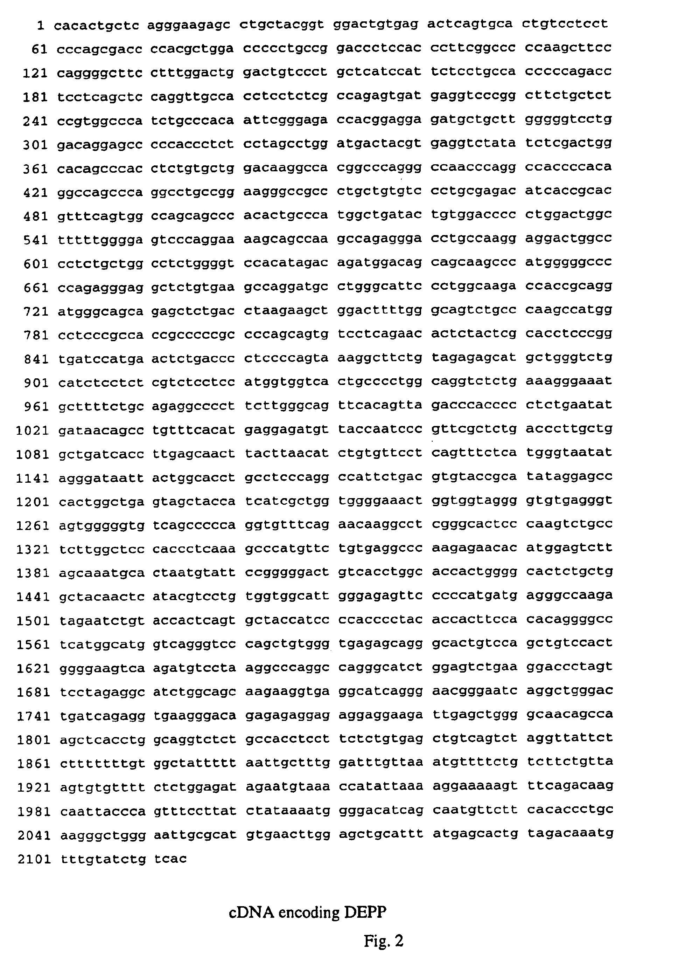

Decidual protein induced by progesterone (DEPP) consists of 212 amino acids and has the following sequence: A cDNA sequence encoding DEPP is shown in Fig. 2 (Seq. Id. No. 4). The function of DEPP is currently unknown.

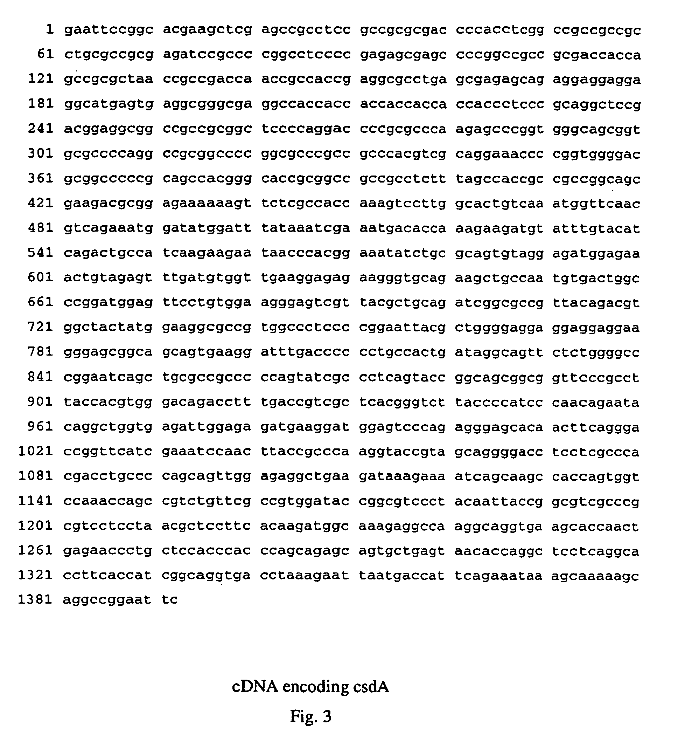

Cold shock domain protein A (csdA) consists of 372 amino acids and has the following sequence: (Seq. Id No. 5). csdA is a member of a family of transcriptional regulators. It is reported to bind and repress the promoter of GM-CSF. A cDNA sequence encoding csdA (Seq. Id. No 6) is shown in Fig. 3.

The surprising expression characteristics of the DEPP, csdA and adrenomedullin genes were uncovered by examination of post mortem anterior cingulate samples from schizophrenic and normal subjects. Samples possessing high quality RNA were utilized for further study. Those skilled in the art are familiar with techniques which may be utilized to determine expression levels. For example, reverse transcriptase assays or DNA microarray analysis can be performed utilizing gene chip technology. Differentially expressed genes can be identified using a number of methods developed in accordance with established principles. Statistical significance of the expression differences between groups of samples may be determined utilizing the t-test, ANOVA or non-parametric tests. In accordance with the present invention, some genes were found to be upregulated in schizophrenic patients while others were found to be downregulated compared to baseline or normal levels. The terms "normal" and "baseline" are used interchangeably herein. Baseline levels are defined using conventional statistical techniques in connection with an analysis of a general population of non-schizophrenics. See, e.g., Example 1 herein. It should be understood, in general, that methods not otherwise specified herein are conducted in accordance with generally accepted principles known to those skilled in the art.

Quantitative rtPCR (Q-PCR) may be conducted on the same samples used for the expression level analysis described above. After conversion of RNA to cDNA using reverse transcriptase, although any conventional PCR technique can be utilized, a preferred technique may be based on the TaqMan® technique (Perkin Elmer Corp., Foster City, CA). In conventional PCR assays, oligonucleotide primers are designed complementary to the 5' and 3'ends of a DNA sequence of interest. During thermal cycling, DNA is heat denatured. The sample is then brought to annealing and extension temperatures in which the primers bind their specific complements and are extended by the addition of nucleotide triphosphates by Taq polymerase. With repeated thermal cycling, the amount of template DNA is amplified. The presence of a dye, such as SybrGreen^{™}, that fluoresces strongly when bound to DNA, allows the real time monitoring of total amount of DNA product in the tube. By measuring this signal, the amplified product can be quantified. The threshold cycle (C_{T}) at which the fluorescent signal is measurably different from the background noise is an accurate measure of the starting amount of cDNA in the tube and hence RNA in the sample. This method allows the quantitation of genes in a complex RNA by targeting specific DNAs. Of the genes initially identified by microarray analysis to be differentially expressed in schizophrenic patients, three, decidual protein induced by progesterone (DEPP), csdA and adrenomedullin, were shown to be differentially regulated in the original and a further independent set of RNA samples.

In one aspect, a method of screening for schizophrenia in a population is provided which includes determining, in members of the population, the magnitude of expression of a gene selected from the group consisting of the gene encoding decidual protein induced by progesterone (DEPP), the gene encoding adrenomedullin and the gene encoding cold shock domain protein A (csdA) in a sample and comparing the magnitude of expression to a baseline magnitude of expression of the gene, wherein increased gene expression indicates the presence of schizophrenia. In another aspect, a method for diagnosing schizophrenia in a host is provided which includes determining the magnitude of expression of a gene selected from the group consisting of the gene encoding decidual protein induced by progesterone (DEPP), the gene encoding adrenomedullin and the gene encoding cold shock domain protein A (csdA) in a sample and comparing the magnitude of expression to a baseline magnitude of expression of the gene, wherein increased gene expression indicates the presence of schizophrenia. In either of the the above screening or diagnosing aspects, the sample may be taken, for example, from the brain, spinal cord, lymphatic fluid, blood, urine or feces. In a preferred embodiment the sample is taken from the anterior cingulated. In another preferred embodiment, the population is human. There are numerous techniques known to those with skill in the art to measure gene expression in a sample. For example, RNA from a cell type or tissue known, or suspected, to express the DEPP, csdA and/or adrenomedullin gene, such as brain, may be isolated and tested utilizing hybridization or PCR techniques such as are described above. The isolated RNA can be derived from primary cell culture from a patient or directly from a biological sample from a patient.

In another aspect, the present invention provides the use of a gene selected from the group consisting of the gene encoding decidual protein induced by progesterone (DEPP), the gene encoding adrenomedullin and the gene encoding cold shock domain protein A (csdA) for the screening of compounds which are useful in the treatment of schizophrenia. The methods entail identifying candidate or test compounds which binds DEPP or adrenomedullin or csdA and/or have a stimulatory or inhibitory effect on the activity or the expression of of DEPP or adrenomedullin or csdA. Preferably, the identification of candidate or test compounds is followed by further determining, which of the compounds that bind DEPP or adrenomedullin or csdA or have a stimulatory or inhibitory effect on the activity or the expression of DEPP or adrenomedullin or csdA, have an effect on schizophrenia in an in vivo assay, such as for instance a transgenic animal as described by the present invention.

In one embodiment of such a detection scheme, a cDNA molecule is synthesized from an RNA molecule of interest (e.g., by reverse transcription of the RNA molecule into cDNA). A sequence within the cDNA is then used as the template for a nucleic acid amplification reaction, such as a PCR amplification reaction, or the like. The nucleic acid reagents used as synthesis initiation reagents (e.g., primers) in the reverse transcription and nucleic acid amplification steps of this method are chosen from among the DEPP, csdA or adrenomedullin gene nucleic acid reagents. Those skilled in the art are familiar with techniques for designing and obtaining suitable primers. See, e.g., Table 1 in Example 2 below. The preferred lengths of such nucleic acid reagents are at least 9-30 nucleotides. For detection of the amplified product, the nucleic acid amplification may be performed using radioactively or non-radioactively labeled nucleotides. Alternatively, enough amplified product may be made such that the product may be visualized by standard ethidium bromide staining or by utilizing any other suitable nucleic acid staining method.

Additionally, it is possible to perform such DEPP, csdA or adrenomedullin gene expression assays "in situ", i.e., directly upon tissue sections (fixed and/or frozen) of patient tissue obtained from biopsies or resections, such that no nucleic acid purification is necessary. Nucleic acid reagents such as those described above may be used as probes and/or primers for such in situ procedures. Alternatively, if a sufficient quantity of the appropriate cells can be obtained, standard Northern analysis can be performed to determine the level of mRNA expression of the DEPP, csdA or adrenomedullin genes.

Regardless of the method used to quantify the expression of the DEPP, csdA and/or adrenomedullin genes, the level of expression in a subject of undefined etiology is compared to a known normal expression level. If the expression level of one, or more than one, of these genes is elevated above the normal or baseline level by about 25%, a diagnosis of schizophrenia may be made or confirmed. Determination of higher levels may be indicative of the severity of the disease.

As demonstrated by the Examples below, one technique for establishing baseline levels may involve real time quantitative PCR. Those skilled in the art are familiar with numerous techniques which may be utilized to test sample populations to obtain statistically sound results. For example, in carrying out this technique, a sample from a population of normal individuals is selected. The sample should be sufficiently diverse in terms of age, sex, social status, geographical distribution, previous drug and medical histories, etc. and of sufficient size to provide a meaningful statistical value. Thus, expression of the DEPP, csdA or adrenomedullin genes is measured in the sample of interest which defines distribution in the normal population. Baseline levels are then assigned. A set of diseased subjects is also assayed to determine validity of the test by comparing results of the diseased sample to those of the normal sample.

In accordance with the present disclosure, symptoms of DEPP, csdA and/or adrenomedullin gene mediated schizophrenia may be ameliorated by decreasing the level of DEPP, csdA and/or adrenomedullin gene expression and/or DEPP, csdA and adrenomedullin gene product activity by using respective DEPP, csdA and adrenomedullin gene sequences in conjunction with well-known antisense, siRNA, gene "knock-out," ribozyme and/or triple helix methods to decrease the level of DEPP, csdA and/or adrenomedullin gene expression. Among the compounds that may exhibit the ability to modulate the activity, expression or synthesis of the DEPP, csdA and/or adrenomedullin gene, including the ability to ameliorate the symptoms of a DEPP, csdA and/or adrenomedullin mediated schizophrenia, are antisense, ribozyme, and triple helix molecules. Such molecules may be designed to reduce or inhibit either unimpaired, or if appropriate, mutant target gene activity. Techniques for the production and use of such molecules are well known to those skilled in the art.

Antisense RNA and DNA molecules act to block the translation of mRNA by hybridizing to target mRNA and preventing protein translation. Antisense approaches involve the design of oligonucleotides that are complementary to a target gene mRNA. The antisense oligonucleotides will bind to the complementary target gene mRNA transcripts and prevent translation. Absolute complementarity, although preferred, is not required.

Double-stranded RNA (dsRNA) can also be used to inhibit gene expression by a mechanism generally known in the art as RNA interference (RNAi), RNAi is described for instance in US Patent 6'506'559 or in Patent Applications WO0244321 or WO0175164. The length of the dsRNA is not crucial for RNAi according to the present invention, however, preferred dsRNAs are such which are generally known in the art as small-inhibitory RNAs (siRNAs). In a preferred embodiment, the siRNAs are short dsRNAs having a length of 19 to 25 nucleotides. Most preferred are dsRNAs having a length of 21 to 23 nucleotides. The dsRNAs may be blunt ended or ligated at or on at least one end with either loops composed of ribonucleotides or deoxyribonucleotides or a chemical synthetic linker (WO00/44895). In a preferred embodiment, the ribonucleic acid contains 3'-end nucleotide overhangs on the antisense strand and / or the sense strands of the dsRNA of at least one ribonucleotide or deoxyribonucleotide, or modified nucleotide. Preferred are overhangs with 1, 2, 3 or 4 nucleotides. The overhangs may contain both ribonucleotide(s) and deoxyribonucleotide(s) which in addition may contain modified sugar moieties. The overhang may be of any sequence, but in a preferred embodiment, the overhang is complementary to the target mRNA strand. In another preferred embodiment the overhang contains at least one UU group or dTdT group. In another preferred embodiment, the overhang on the antisense strand has the penultimate overhanging nucleotide complementary to the mRNA target strand. Preferably, such an overhang is a 2-nucleotides overhang. In a further preferred embodiment, the overhang is composed of 4 Us. In another preferred embodiment, the extreme 3'-position of the siRNA is a hydroxyl group. Additionally, the 5'-end may be a hydroxyl or phosphate group.

A sequence "complementary" to a portion of an RNA, as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

In one embodiment, oligonucleotides complementary to coding or non-coding regions of the DEPP, csdA and/or adrenomedullin genes could be used in an antisense or RNAi approach to inhibit translation of endogenous DEPP, csdA and/or adrenomedullin mRNA. Based upon the sequences presented in Figs. 1-3 or upon allelic or homologous genomic and/or DNA sequences, one of skill in the art can easily choose and synthesize any of a number of appropriate antisense or siRNA molecules for use in accordance with the present invention. Antisense nucleic acids should be at least six nucleotides in length, and are preferably oligonucleotides ranging from 6 to about 50 nucleotides in length. In certain preferred aspects the oligonucleotide length is from about 8 to about 30 nucleotides.

Suitable antisense oligonucleotides herein encompass modified oligonucleotides which may exhibit enhanced stability, targeting or which otherwise exhibit enhanced therapeutic effectiveness. Examples of modified oligonucleotides include those where (1) at least two nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Examples of synthetic internucleoside linkages are phosphorothioates, alkylphosphonates, phosphorodithioates, phosphate esters, alkylphosphonothioates, phosphoramidates, carbamates, phosphate triesters, acetamidates, peptides, and carboxymethyl esters. Modified oligonucleotides may also have covalently modified bases and/or sugars. For example, oligonucleotides having backbone sugars which are covalently attached to low molecular weight organic groups other than a hydroxyl group at the 3' position and other than a phosphate group at the 5' position. Thus modified oligonucleotides may include a 2'-O-alkylated ribose group. In addition, modified oligonucleotides may include sugars such as arabinose instead of ribose. Modified oligonucleotides also can include base analogs such as C-5 propyne modified bases.

Antisense oligonucleotides or siRNA molecules may be synthesized by standard techniques known in the art, e.g., by use of an automated DNA synthesizer (such as are commercially available from Biosearch, Applied Biosystems, etc.). As examples, phosphorothioate oligonucleotides may be synthesized by the method of Stein, et al. (1988, Nucl. Acids Res. 16, 3209), methylphosphonate oligonucleotides can be prepared by use of controlled pore glass polymer supports (Sarin, et al., 1988, Proc. Natl. Acad. Sci. U.S.A. 85, 7448-7451), etc.

While antisense nucleotides complementary to the target gene coding region sequence could be used, those complementary to the transcribed, untranslated region are most preferred. A preferred site is the region encompassing the translation initiation or termination codon of the open reading frame (ORF) of the gene. Those with skill in the art are well aware of various suitable initiation or termination codons in both eukaryotes and prokaryotes.

Antisense or siRNA molecules may be delivered to cells that express the target gene in vivo or in vitro. A number of methods have been developed for delivering antisense DNA or RNA or siRNAi to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically. A preferred technique involves constructing a vector which incorporates a strong promoter to provide high expression and good yield of antisense or siRNA oligonucleotides at the target site. The use of such a construct to transfect target cells in the patient results in the transcription of sufficient amounts of single stranded RNAs that will form complementary base pairs with the endogenous target gene transcripts and thereby prevent translation of the target gene mRNA or results in the transcription of sufficient amount of single-stranded RNAs complenemtary to each other that form a dsRNA capable of inhibiting gene expression by RNAi. For example, a vector can be introduced such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods known to those in the art. Vectors can be, e.g., plasmid, viral, or others typically used for replication and expression in mammalian cells. It should be understood that expression of the sequence encoding the antisense RNA can be by any promoter known in the art to act in mammalian, preferably human cells. Such promoters can be inducible or constitutive. Any type of plasmid, cosmid, YAC, BAC or viral vector can be used to prepare the recombinant DNA construct which can be introduced directly into the tissue site. Alternatively, viral vectors can be used that selectively infect the desired tissue, in which case administration may be accomplished by another route (e.g., systemically).

Also disclosed is the use of one or more antisense or siRNA molecules that specifically inhibit the expression of DEPP, csdA and/or adrenomedullin genes for the manufacture of a medicament useful in the treatment of schizophrenia.

Ribozyme molecules designed to catalytically cleave target gene mRNA transcripts can also be used to prevent or reduce translation of DEPP, csdA or adrenomedullin target gene mRNA and, therefore, expression of target gene product. (See, e.g., PCT International Publication WO90/11364, published Oct. 4, 1990; Sarver, et: al., 1990, Science 247, 1222-1225). Ribozymes are enzymatic RNA molecules capable of catalyzing the specific cleavage of RNA. The mechanism of ribozyme action involves sequence specific hybridization of the ribozyme molecule to complementary target RNA, followed by an endonucleolytic cleavage event. The composition of ribozyme molecules must include one or more sequences complementary to the target gene mRNA, and must include the well known catalytic sequence responsible for mRNA cleavage. For this sequence, see, e.g., U.S. Pat. No. 5,093,246.

Ribozymes that cleave mRNA at site specific recognition sequences can be used to destroy target gene mRNAs. For example, hammerhead ribozymes may be utilized to cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art. Preferably, the ribozyme is engineered so that the cleavage recognition site is located near the 5' end of the target gene mRNA, i.e., to increase efficiency and minimize the intracellular accumulation of non-functional protein fragments. Suitable ribozymes also include RNA endoribonucleases such as the one that occurs naturally in Tetrahymena thermophila (known as the IVS, or L-19 IVS RNA). This type of ribozymes have an eight base pair active site which hybridizes to a target RNA sequence to effect cleavage of the target RNA.

As in the antisense approach, the ribozymes can be composed of modified oligonucleotides (e.g., for improved stability, targeting, etc.) and should be delivered to cells that express the target gene in vivo. A preferred method of delivery involves using a DNA construct "encoding" the ribozyme under the control of a strong constitutive promoter, so that transfected cells will produce sufficient quantities of the ribozyme to destroy endogenous DEPP csdA and/or adrenomedullin gene messages and inhibit translation. Because ribozymes, unlike antisense molecules, are catalytic, a lower intracellular concentration is required for efficiency.

Alternatively, endogenous DEPP, csdA and/or adrenomedullin gene expression can be reduced by targeting deoxyribonucleotide sequences complementary to the regulatory region of the target DEPP, csdA and/or adrenomedullin genes (i.e., the target gene promoter and/or enhancers) to form triple helical structures that prevent transcription of the target gene in target cells in the body. Nucleic acid molecules to be used in triplex helix formation for the inhibition of transcription should be single stranded and composed of deoxynucleotides. The base composition of these oligonucleotides must be designed to promote triple helix formation via Hoogsteen base pairing rules, which generally require sizeable stretches of either purines or pyrimidines to be present on one strand of a duplex. Nucleotide sequences may be pyrimidine-based, which will result in TAT and CGC⁺ triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex.

Alternatively, the potential sequences that can be targeted for triple helix formation may be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

Also disclosed is the use of one or more ribozyme or nucleic acid molecule promoting triple helix formation that specifically inhibit the expression of DEPP, csdA and/or adrenomedullin genes for the manufacture of a medicament useful in the treatment of schizophrenia.

Anti-sense RNA and DNA, siRNA, ribozyme, and triple helix molecules described herein may be prepared by any method known in the art for the synthesis of DNA and RNA molecules, as discussed above. These include techniques for chemically synthesizing oligodeoxyribonucleotides and oligoribonucleotides well known in the art such as for example solid phase phosphoramidite chemical synthesis. Alternatively, RNA molecules may be generated by in vitro and in vivo transcription of DNA sequences encoding the antisense RNA molecule. Such DNA sequences may be incorporated into a wide variety of vectors that incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

In another aspect, the present invention provides the use of a gene selected from the group consisting of the gene encoding decidual protein induced by progesterone (DEPP), the gene encoding adrenomedullin and the gene encoding cold shock domain protein A (csdA) as a biomarker for schizophrenia. In addition, this invention provides methods for screening a subject to determine if biomarkers for schizophrenia are present in order to confirm a diagnosis made on purely clinical grounds. This invention also provides methods for monitoring the severity or progression of the schizophrenia in an individual.

A method of modulating DEPP, csdA and/or adrenomedullin to treat schizophrenia is provided by exposing neutralizing antibodies to DEPP, csdA and/or adrenomedullin proteins. By providing for controlled exposure to such antibodies, protein abundances/activities can be controllably modified. For example, antibodies to suitable epitopes on protein surfaces may decrease the abundance, and thereby indirectly decrease the activity, of the wild-type active form of DEPP, csdA and/or adrenomedullin proteins by aggregating active forms into complexes with less or minimal activity as compared to the wild-type unaggregated wild-type form. Alternately, antibodies may directly decrease protein activity by, e.g., interacting directly with active sites or by blocking access of substrates to active site. In either case, antibodies can be raised against specific protein species and their effects screened. The effects of the antibodies can be assayed and suitable antibodies selected that lower the target protein species concentration and/or activity. Such assays involve introducing antibodies into a cell or surrounding media, and assaying the concentration of the wild-type amount or activities of the target protein by standard means (such as immunoassays) known in the art. The net activity of the wild-type form can be assayed by assay means appropriate to the known activity of the target protein.

Thus, in another aspect, the use of an antibody or several antibodies that specifically bind an epitope of DEPP, csdA and/or adrenomedullin proteins for the manufacture of a medicament useful in the treatment of schizophrenia is also disclosed.

Antibodies can be introduced into cells in numerous ways, including, for example, microinjection of antibodies into a cell (Morgan et al., 1988, Immunology Today 9:84-86) or transforming hybridoma mRNA encoding a desired antibody into a cell (Burke et al., 1984, Cell 36:847-858). In a further technique, recombinant antibodies can be engineered and ectopically expressed in a wide variety of non-lymphoid cell types to bind to target proteins as well as to block target protein activities. Preferably, expression of the antibody is under control of a controllable promoter, such as the Tet promoter. A first step is the selection of a particular monoclonal antibody with appropriate specificity to the target protein. Then sequences encoding the variable regions of the selected antibody can be cloned into various engineered antibody formats, including, for example, whole antibody, Fab fragments, Fv fragments, single chain Fv fragments (VH and VL regions united by a peptide linker) ("ScFv" fragments), diabodies (two associated ScFv fragments with different specificities), and so forth. Intracellularly expressed antibodies of the various formats can be targeted into cellular compartments by expressing them as fusions with the various known intracellular leader sequences.

Methods for the production of antibodies capable of specifically recognizing one or more DEPP, csdA and or adrenomedullin gene product epitopes or epitopes of conserved variants or peptide fragments of the DEPP, csdA and/or adrenomedullin gene products are well known in the art. Such antibodies may include, but are not limited to, polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

Such antibodies may also be used, for example, in the detection of a DEPP, csdA and/or adrenomedullin gene product in an biological sample and may, therefore, be utilized as part of a diagnostic or prognostic technique whereby patients may be tested for abnormal levels of DEPP, csdA and/or adrenomedullin gene products, and/or for the presence of abnormal forms of such gene products. Such antibodies may also be utilized in conjunction with, for example, compound screening schemes, for the evaluation of the effect of test compounds on DEPP, csdA and/or adrenomedullin gene product levels and/or activity.

For the production of antibodies against a DEPP, csdA and/or adrenomedullin gene product, various host animals may be immunized by injection with a DEPP, csdA and/or adrenomedullin gene product, or a portion thereof. Such host animals may include, but are not limited to rabbits, mice, and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum.

Polyclonal antibodies are heterogeneous populations of antibody molecules derived from the sera of animals immunized with an antigen, such as a DEPP, csdA and/or adrenomedullin gene product, or an antigenic functional derivative thereof. For the production of polyclonal antibodies, host animals such as these described above, may be immunized by injection with DEPP, csdA and/or adrenomedullin supplemented with adjuvants as also described above.

Monoclonal antibodies, which are homogeneous populations of antibodies to a particular antigen, may be obtained by any technique that provides for the production of antibody molecules by continuous cell lines in culture. These include, but are not limited to, the hybridoma technique of Kohler and Milstein, (1975, Nature 256, 495-497; and U.S. Pat. No. 4,376,110), the human B-cell hybridoma technique (Kosbor et al., 1983, Immunology Today 4, 72; Cole et al., 1983, Proc. Natl. Acad. Sci. USA 80, 2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb may be cultivated in vitro or in vivo.

In addition, techniques developed for the production of "chimeric antibodies" (Morrison, et al., 1984, Proc. Natl. Acad. Sci., 81, 6851-6855; Neuberger, et al., 1984, Nature 312, 604-608; Takeda, et al., 1985, Nature, 314, 452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Techniques have also been developed for the production of humanized antibodies. (See, e.g., Queen, U.S. Pat. No. 5,585,089,). An immunoglobulin light or heavy chain variable region consists of a "framework" region interrupted by three hypervariable regions, referred to as complementarity determining regions (CDRs). The extent of the framework region and CDRs have been precisely defined (see, e.g., "Sequences of Proteins of Immunological Interest", Kabat, E. et al., U.S. Department of Health and Human Services (1983)). Briefly, humanized antibodies are antibody molecules from non-human species having one or more CDRs from the non-human species and a framework region from a human immunoglobulin molecule. Alternatively, techniques described for the production of single chain antibodies (U.S. Pat. No. 4,946,778; Bird, 1988, Science 242, 423-426; Huston, et al., 1988, Proc. Natl. Acad. Sci. USA 85, 5879-5883; and Ward, et al., 1989, Nature 334, 544-546) can be adapted to produce single chain antibodies against DEPP, csdA and/or adrenomedullin gene products. Single chain antibodies are formed by linking the heavy and light chain fragments of the Fv region via an amino acid bridge, resulting in a single chain polypeptide.

Antibody fragments that recognize specific epitopes may be generated by known techniques. For example, such fragments include but are not limited to: the F(ab')₂ fragments, which can be produced by pepsin digestion of the antibody molecule and the Fab fragments, which can be generated by reducing the disulfide bridges of the F(ab')₂ fragments. Alternatively, Fab expression libraries may be constructed (Huse, et al., 1989, Science, 246, 1275-1281) to allow rapid and easy identification of monoclonal Fab fragments with the desired specificity.

Antibodies, or fragments of antibodies, such as those described, above, may be used to quantitatively or qualitatively detect the presence of DEPP, csdA and/or adrenomedullin gene products or conserved variants or peptide fragments thereof. This can be accomplished, for example, by immunofluorescence techniques employing a fluorescently labeled antibody coupled with light microscopic, flow cytometric, or fluorometric detection.

The antibodies (or fragments thereof) useful in the present invention may be employed histologically, as in immunofluorescence or immunoelectron microscopy, for in situ detection of DEPP, csdA and/or adrenomedullin gene products, conserved variants or peptide fragments thereof. In situ detection may be accomplished by removing a histological specimen from a patient, and applying thereto a labeled antibody that binds to a DEPP, csdA and/or adrenomedullin polypeptide. The antibody (or fragment) is preferably applied by overlaying the labeled antibody (or fragment) onto a biological sample. Through the use of such a procedure, it is possible to determine not only the presence of DEPP, csdA and/or adrenomedullin, conserved variants or peptide fragments, but also its distribution in the examined tissue. Using the present invention, those of ordinary skill will readily recognize that any of a wide variety of histological methods (such as staining procedures) can be modified in order to achieve in situ detection of DEPP, csdA and/or adrenomedullin.

Immunoassays for DEPP, csdA and/or adrenomedullin, conserved variants, or peptide fragments thereof will typically comprise incubating a sample, such as a biological fluid, a tissue extract, freshly harvested cells, or lysates of cells in the presence of a detectably labeled antibody capable of identifying DEPP, csdA and/or adrenomedullin, conserved variants or peptide fragments thereof, and detecting the bound antibody by any of a number of techniques well-known in the art. The biological sample may be brought in contact with and immobilized onto a solid phase support or carrier, such as nitrocellulose, that is capable of immobilizing cells, cell particles or soluble proteins. The support may then be washed with suitable buffers followed by treatment with the detectably labeled DEPP, csdA and/or adrenomedullin specific antibodies. The solid phase support may then be washed with the buffer a second time to remove unbound antibody. The amount of bound label on the solid support may then be detected by conventional means.

One of the ways in which the DEPP, csdA and/or adrenomedullin -specific antibodies can be detectably labeled is by linking the same to an enzyme, such as for use in an enzyme immunoassay (EIA). The enzyme, which is bound to the antibody, will react with an appropriate substrate, preferably a chromogenic substrate, in such a manner as to produce a chemical moiety that can be detected, for example, by spectrophotometric, fluorimetric or by visual means. Enzymes that can be used to detectably label the antibody are well known. The detection can be accomplished by colorimetric methods that employ a chromogenic substrate for the enzyme. Detection may also be accomplished by visual comparison of the extent of enzymatic reaction of a substrate in comparison with similarly prepared standards. Detection may also be accomplished using any of a variety of other immunoassays. For example, by radioactively labeling the antibodies or antibody fragments, it is possible to detect DEPP, csdA and/or adrenomedullin through the use of a radioimmunoassay (RIA). The radioactive isotope can be detected by such means as the use of a gamma counter or a scintillation counter or by autoradiography. It is also possible to label the antibody with a fluorescent compound. When the fluorescently labeled antibody is exposed to light of the proper wavelength, its presence can then be detected due to fluorescence. Among the most commonly used fluorescent labeling compounds are green fluorescent protein, fluorescein isothiocyanate, rhodamine, phycoerythrin, phycocyanin, allophycocyanin, o-phthaldehyde and fluorescamine. The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA). The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester. Likewise, a bioluminescent compound may be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling include luciferin, luciferase and aequorin.

The present invention contemplates production of animal models that have abnormal expression levels of DEPP, csdA and/or adrenomedullin to study the effects of increased or decreased levels of these proteins on such animals. Such animals provide test subjects for determining the effects of therapeutic or potentially therapeutic compounds on schizophrenia. Accordingly, the DEPP, csdA and/or adrenomedullin gene products can be expressed in transgenic animals. Animals of any species, including, but not limited to, mice, rats, rabbits, guinea pigs, pigs, mini-pigs, goats, sheep, and non-human primates, e.g., baboons, monkeys, and chimpanzees may be used to generate DEPP, csdA and/or adrenomedullin transgenic animals. The term "transgenic," as used herein, refers to animals expressing DEPP, csdA and/or adrenomedullin gene sequences from a different species (e.g., mice expressing human DEPP, csdA and/or adrenomedullin gene sequences), as well as animals that have been genetically engineered to overexpress endogenous (i.e., same species) DEPP, csdA and/or adrenomedullin sequences or animals that have been genetically engineered to no longer express endogenous DEPP, csdA and/or adrenomedullin gene sequences (i.e., "knockout" animals), and their progeny.

Any technique known in the art may be used to introduce DEPP, csdA and/or adrenomedullin genes into animals to produce the founder lines of transgenic animals. Such techniques include, but are not limited to pronuclear microinjection (Hoppe and Wagner, 1989, U.S. Pat. No. 4,873,191); retrovirus mediated gene transfer into germ lines (Van der Putten, et al., 1985, Proc. Natl. Acad. Sci., USA 82, 6148-6152); gene targeting in embryonic stem cells (Thompson, et al., 1989, Cell 56, 313-321); electroporation of embryos (Lo, 1983, Mol. Cell. Biol. 3, 1803-1814); and sperm-mediated gene transfer (Lavitrano et al., 1989, Cell 57, 717-723) (For a review of such techniques, see Gordon, 1989, Transgenic Animals, Intl. Rev. Cytol. 115, 171-229). Any technique known in the art may be used to produce transgenic animal clones containing a DEPP, csdA and/or adrenomedullin transgene, for example, nuclear transfer into enucleated oocytes of nuclei from cultured embryonic, fetal or adult cells induced to quiescence (Campbell, et al., 1996, Nature 380, 64-66; Wilmut, et al., 1997,Nature 385, 810-813).

The present invention contemplates the use of transgenic animals that carry an DEPP, csdA and/or adrenomedullin transgene in all their cells, as well as animals that carry the transgene in some, but not all their cells, i.e., mosaic animals. The transgene may be integrated as a single transgene or in concatamers, e.g., head-to-head tandems or head-to-tail tandems. The transgene may also be selectively introduced into and activated in a particular cell type by following, for example, the teaching of Lasko et al. (Lasko, et al., 1992, Proc. Natl. Acad. Sci. USA 89, 6232-6236). The regulatory sequences required for such a cell-type specific activation will depend upon the particular cell type of interest, and will be apparent to those of skill in the art. When it is desired that the DEPP, csdA and/or adrenomedullin transgene be integrated into the chromosomal site of the endogenous DEPP, csdA and/or adrenomedullin gene, gene targeting is preferred. Briefly, when such a technique is to be utilized, vectors containing some nucleotide sequences homologous to the endogenous DEPP, csdA and/or adrenomedullin gene are designed for the purpose of integrating, via homologous recombination with chromosomal sequences, into and disrupting the function of the nucleotide sequence of the endogenous DEPP, csdA and/or adrenomedullin gene. The transgene may also be selectively introduced into a particular cell type, thus inactivating the endogenous DEPP, csdA and/or adrenomedullin gene in only that cell type, by following, for example, the teaching of Gu, et al., 1994, Science 265, 103-106. The regulatory sequences required for such a cell-type specific inactivation will depend upon the particular cell type of interest, and will be apparent to those of skill in the art.

As mentioned above, transgenic knockout animals are also contemplated herein. In such transgenic animals DEPP, csdA and/or adrenomedullin gene expression is undetectable or insignificant. Any technique known in the art may be used to produce such transgenic knockout animals. This may be achieved by a variety of mechanisms, e.g., alteration of any or all of the DEPP, csdA and/or adrenomedullin genes by, e.g., introduction of a disruption of the coding sequence, e.g., insertion of one or more stop codons, insertion of a DNA fragment, etc., deletion of regulatory or coding sequence, substitution of stop codons for coding sequence, etc. The transgenic animals may be either homozygous or heterozygous for the alteration. A functional knock-out may also be achieved by the introduction of an anti-sense construct that blocks expression of the native genes. Knockouts also include conditional knockouts such as where alteration of the target gene occurs upon exposure of the animal to a substance that promotes target gene alteration, introduction of an enzyme that promotes recombination at the target gene site, or other method for directing the target gene alteration postnatally.

Once transgenic animals have been generated, the expression of the recombinant DEPP, csdA and/or adrenomedullin gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques described above and those that include but are not limited to Northern blot analysis of tissue samples obtained from the animal, in situ hybridization analysis, and RT-PCR (reverse transcriptase PCR). Samples of DEPP, csdA and/or adrenomedullin gene-expressing tissue, may also be evaluated immunocytochemically using antibodies specific for the DEPP, csdA and/or adrenomedullin transgene product.

Through use of the subject transgenic animals or cells derived therefrom, one can identify ligands or substrates that modulate phenomena associated with schizophrenia, e.g., behavioral phenomena. A wide variety of assays may be used for this purpose, including behavioral studies, determination of the localization of drugs after administration and the like. Depending on the particular assay, whole animals may be used, or cells derived therefrom. Cells may be freshly isolated from an animal, or may be immortalized in culture. Cells of particular interest are derived from neural tissue.

The term " therapeutic agent" as used herein describes any molecule, e.g. protein, carbohydrate, metal or organic compound, with the capability of affecting the molecular and clinical phenomena associated with schizophrenia. Generally a plurality of assay mixtures may be run in parallel with different agent concentrations to obtain a differential response to the various concentrations. Typically, one of these concentrations serves as a negative control, i.e. at zero concentration or below the level of detection.

Candidate therapeutic agents encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate therapeutic agents often comprise cyclical carbon or heterocyclic structures and/or aromatic or polyaromatic structures substituted with one or more of the above functional groups. Candidate therapeutic agents are also found among biomolecules including, but not limited to: peptides, saccharides, fatty acids, steroids, purines, pyrimidines, derivatives, structural analogs or combinations thereof.

As mentioned above, antibodies specific for DEPP, csdA and/or adrenomedullin gene products may be used in screening immunoassays, particularly to detect the level of such products in a cell or sample. The number of cells in a sample will generally be at least about 10³, usually at least 10⁴ more usually at least about 10⁵. The cells may be dissociated, in the case of solid tissues, or tissue sections may be analyzed. Alternatively a lysate of the cells may be prepared. For example, detection may utilize staining of cells or histological sections, performed in accordance with conventional methods. The antibodies of interest are added to the cell sample, and incubated for a period of time sufficient to allow binding to the epitope, usually at least about 10 minutes. The antibody may be labeled with radioisotopes, enzymes, fluorescers, chemiluminescers, or other labels for direct detection. Alternatively, a second stage antibody or reagent is used to amplify the signal. Such reagents are well known in the art. For example, the primary antibody may be conjugated to biotin, with horseradish peroxidase-conjugated avidin added as a second stage reagent. Final detection uses a substrate that undergoes a color change in the presence of the peroxidase. The absence or presence of antibody binding may be determined by various methods, including flow cytometry of dissociated cells, microscopy, radiography, scintillation counting, etc.

A number of assays are known in the art for determining the effect of a drug on animal behavior and other phenomena associated with schizophrenia. Some examples are provided, although it will be understood by one of skill in the art that many other assays may also be used. The subject animals may be used by themselves, or in combination with control animals.

The screen using the transgenic animals of the invention can employ any phenomena associated with schizophrenia that can be readily assessed in an animal model. The screening for schizophrenia can include assessment of phenomena including, but not limited to: 1) analysis of molecular markers (e.g., levels of expression of DEPP, csdA and/or adrenomedullin gene products in brain tissue; presence/absence in brain tissue of various DEPP, csdA and/or adrenomedullin splice variants; 2) assessment of behavioral symptoms associated with memory and learning; and 3) detection of neurodegeneration. Preferably, the screen will include control values (e.g., the level of DEPP, csdA and/or adrenomedullin production in the test animal in the absence of test compound(s)). Test substances which are considered positive, i.e., likely to be beneficial in the treatment of schizophrenia, will be those which have a substantial effect upon a schizophrenia associated phenomenon (e.g., test agents that are able to normalize erratic or abnormal behavior or that reduce the level of DEPP, csdA and/or adrenomedullin production to within the normal range).

The present invention also encompasses the use of cell-based assays or cell-lysate assays (e.g., in vitro transcription or translation assays) to screen for compounds or compositions that modulate DEPP, csdA and/or adrenomedullin gene expression. To this end, constructs containing a reporter sequence linked to a regulatory element of the any or all of the genes encoding DEPP, csdA and/or adrenomedullin can be used in engineered cells, or in cell lysate extracts, to screen for compounds that modulate the expression of the reporter gene product at the level of transcription. For example, such assays could be used to identify compounds that modulate the expression or activity of transcription factors involved in expression of the genes encoding DEPP, csdA and/or adrenomedullin, or to test the activity of triple helix polynucleotides. Alternatively, engineered cells or translation extracts can be used to screen for compounds (including antisense and ribozyme constructs) that modulate the translation of DEPP, csdA and/or adrenomedullin mRNA transcripts, and therefore, affect expression of the DEPP, csdA and/or adrenomedullin gene products. Thus, regulatory regions such as a promoter are operatively linked to a gene encoding a reporter molecule such as green fluorescent protein (GFP), luciferase and the like, to create a reporter construct which is regulated by the DEPP, csdA and/or adrenomedullin regulatory sequences. The gene construct is then transfected into a desired cell such as a neuronal cell. The baseline expression levels of the reporter molecule are then calculated using conventional methods. The cell is then exposed to a test compound and the level of expression of the reporter molecule is determined and compared to the baseline levels. A compound which reduces the amount of reporter expression is a candidate for the treatment of schizophrenia. A second screening procedure may then be instituted to determine whether the compound affects the level of expression of the gene(s) encoding DEPP, csdA and/or adrenomedullin by measuring the amount of RNA or protein from the native gene(s). Construction of neuronal cells incorporating a reporter gene for determining the effect of compounds on expression is known, e.g., see, Asselbergs et al., Nucleic Acids Res 27:1826-33(1998).

Antisense compounds, ribozymes, antibodies and other DEPP, csdA and/or adrenomedullin knockout devices or modulators (collectively referred to for convenience as the "modulators") described herein may be admixed, encapsulated, conjugated or otherwise associated with other molecules, molecular structures or mixtures of compounds, as for example, liposomes, receptor targeted molecules, oral, rectal, topical, or other formulations, for assisting in uptake, distribution and/or absorption. Those skilled in the art are familiar with a myriad of techniques to produce such devices.

It is contemplated that the modulators may encompass any pharmaceutically acceptable salts, esters, or salts of such esters, or any other compound which, upon administration to an animal including a human, is capable of providing (directly or indirectly) the biologically active metabolite or residue thereof. Accordingly, for example, the disclosure is also drawn to prodrugs and pharmaceutically acceptable salts of the compounds of the invention, pharmaceutically acceptable salts of such prodrugs, and other bioequivalents. The term "pharmaceutically acceptable salts" refers to physiologically and pharmaceutically acceptable salts of the compounds of the invention: i.e., salts that retain the desired biological activity of the parent compound and do not impart undue toxicological effects thereto. Such compounds may be prepared according to conventional methods by one of skill in the art. (Berge et al., "Pharmaceutical Salts," J. of Pharma Sci., 1977, 66, 1-19). The term "prodrug" indicates a therapeutic agent that is prepared in an inactive form that is converted to an active form (i.e., drug) within the body or cells thereof by the action of endogenous enzymes or other chemicals and/or conditions. In particular, prodrug versions of the oligonucleotides may be prepared as SATE [(S-acetyl-2-thioethyl)phosphate] derivatives according to the methods disclosed in WO 93/24510 to Gosselin et al., or in WO 94/26764 to Imbach et al.

The modulators herein can be utilized for diagnostics, therapeutics, prophylaxis and as research reagents and kits. For therapeutics, an animal, preferably a human, suspected of having a schizophrenic disease or disorder which can be treated by modulating the expression of one or more DEPP, csdA and/or adrenomedullin regulated genes, is treated by administering modulators in accordance with this invention. The modulators can be utilized in pharmaceutical compositions by adding an effective amount of one or more modulators to a suitable pharmaceutically acceptable diluent or carrier. Those skilled in the art are familiar with numerous techniques and formulations utilized to compound pharmaceutical compositions. The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of liquids, powders or aerosols, including by nebulizer, intratracheal, intranasal, enteral, epidermal and transdermal), oral, sublingual, buccal or parenteral. Parenteral administration includes intravenous, intraarterial, subcutaneous, intraperitoneal or intramuscular injection or infusion; intramedullary or intracranial, e.g., intrathecal or intraventricular, administration. Oligonucleotides with at least one 2'-O-methoxyethyl modification may be useful for oral administration.

Pharmaceutical compositions for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Compositions and formulations for oral administration include powders or granules, suspensions or solutions in water or non-aqueous media, capsules, sachets, troches or tablets. Thickeners, flavoring agents, diluents, emulsifiers, dispersing aids or binders may be desirable. Compositions for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients. Pharmaceutical compositions of the present invention include, but are not limited to, solutions, emulsions, suspensions, foams and liposome-containing formulations. These compositions may be generated from a variety of components that include, but are not limited to, preformed liquids, self-emulsifying solids and self-emulsifying semisolids, according to conventional methods, by one of skill in the art.

The pharmaceutical formulations of the present disclosure, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product. Further details on techniques for formulation and administration of numerous dosage forms may be found in the latest edition of Remington's Pharmaceutical Sciences (Maack Publishing Co., Easton, Pa.). The compositions may be administered alone or in combination with at least one other agent, such as stabilizing compound, which may be administered in any sterile, biocompatible pharmaceutical carrier, including, but not limited to, saline, buffered saline, dextrose, and water. The compositions may be administered to a patient alone, or in combination with other agents, drugs or hormones.

Pharmaceutical formulations suitable for parenteral administration may be formulated in aqueous solutions, preferably in physiologically compatible buffers such as Hanks' solution, Ringer's solution, or physiologically buffered saline. Aqueous injection suspensions may contain substances that increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers may also be used for delivery. Optionally, the suspension may also contain suitable stabilizers or agents that increase the solubility of the compounds to allow for the preparation of highly concentrated solutions. For topical or nasal administration, penetrants appropriate to the particular barrier to be permeated are used in the formulation. Such penetrants are generally known in the art.

Pharmaceutical compositions suitable for use in the invention include compositions wherein the modulators are contained in an effective amount to achieve the intended purpose. The determination of an effective dose is well within the capability of those skilled in the art. For any compound, the therapeutically effective dose can be estimated initially either in cell culture assays, e.g., of neoplastic cells, or in animal models, usually mice, rabbits, dogs, or pigs. The animal model may also be used to determine the appropriate concentration range and route of administration. Such information can then be used to determine useful doses and routes for administration in humans. A therapeutically effective dose refers to that amount of active ingredient, which ameliorates, partially or completely, the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between toxic and therapeutic effects is the therapeutic index, and it can be expressed as the ratio, LD50/ED50. Pharmaceutical compositions that exhibit large therapeutic indices are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage form employed, sensitivity of the patient, and the route of administration.

The exact dosage will be determined by the practitioner in light of factors related to the subject that require treatment Dosage and administration are adjusted to provide sufficient levels of the modulators to maintain the desired effect. Factors which may be taken into account include the severity of the disease state, general health of the subject, age, weight, and gender of the subject, diet, time and frequency of administration, drug combination(s), reaction sensitivities, and tolerance/response to therapy- Long-acting pharmaceutical compositions may be administered every 3 to 4 days, every week, or once every two weeks depending on half-life and clearance rate of the particular formulation. Normal dosage amounts may vary from 0.1 to 100,000 micrograms, up to a total dose of about 1 g per kilogram, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

The following examples are included for purposes of illustration and should not be construed as limiting the present invention.

### EXAMPLE 1

### DNA Microarray Analysis

Human anterior cingulate samples are obtained from 10 normal and 10 schizophrenic deceased subjects (Maryland Psychiatric Research Clinic, Baltimore, Maryland). Good quality RNA is obtained from 9 normal ("N1") and 7 schizophrenic ("S1") samples.

The microarray analysis is performed essentially as follows. Briefly, 5µg or less total RNA is used to synthesize cDNA which is then used as a template to generate biotinylated cRNA. 15 to 30 µg labeled RNA is obtained and hybridized to Affymetrix (Santa Clara, CA) the Human Genome U95Av2 Array of the GeneChip® Human Genome U95 Set (HG-U95Av2 contains ≈12,000 sequences of full length genes) in accordance with the protocols found in the GeneChip® technical manual. Each sample is profiled in duplicate. After sample hybridization, microarrays are washed and scanned with a laser scanner.

The images obtained are used to generate absolute text files for analysis using Affymetrix GeneChip® Gene Expression Analysis Algorithms version 4. Differentially expressed genes between the normal and schizophrenic derived samples are ranked using a pattern recognition algorithm developed in accordance with established principles which generated a score for each gene being compared. The following three conditions are required for a score (equal to the mean fold change) to be generated: (1) t-test p-value<0.5%; (2) average fold-change>1.5; (3) maximum mean AvgDiff (expression levels on an Affymetrix chip)>200. If one or more of the above conditions is not met by a gene in comparison, the score assigned is zero. Several candidate genes are found to be differentially expressed in schizophrenic patients when compared to normal.

### EXAMPLE 2

### Real Time Quantitative PCR Confirmation Of Differentially Regulated Genes

Probe pairs for real time quantitative PCR (Q-PCR) are designed for 38 altered genes identified in Example 1. Affymetrix provides a file of sequences from which the probes on the chip are derived. From this file, the sequences corresponding to these 38 altered genes are obtained, and the probe pairs are prepared. Where a good pair of primers cannot be obtained from Affymetrix sequence, a longer sequence can be obtained from Ref Seq. (See Pruitt KD, Maglott DR Nucleic Acids Res 2001 Jan 1;29(1):137-140;Introducing RefSeq and LocusLink: curated human genome resources at the NCBI., Pruitt KD, Katz KS, Sicotte H, Maglott DR Trends Genet. 2000 Jan;16(1):44-47) with a good BLAST score against the Affymetrix sequence and the primers are designed from that sequence. The sequences of the probe pairs are presented in Table 1.

**Table 1**

| Affymetrix Gene ID | Primer name | Sequence 5'-3' | Primer name | Sequence 5'-3' |
|---|---|---|---|---|
| GAPDH | GAPDH5F | CAGGGCTGCTTTTAACTCTGGTA (Seq. Id. 7) | GAPDH5 R | GGGTGGAATCATATTGGAACATGT (Seq. Id. 8) |
| 1622_at | 1622p2F | TTTTAATCTCTCGACTGAATGGACTTT (Seq. Id. 9) | 1622p2R | CACAACTCATCCCCTCTTGTGTAC (Seq. Id. 10) |
| 1731_at | 1731 for | TGTGTTTTCAGCAAATTCCAGATT (Seq. Id. 11) | 1731 rev | TGTCATCATAAAAATAGAAAGTAGG AAAGATT (Seq. Id. 12) |
| 32279_at | 32279for | TTCTGTTCTCAAATGCTAAATGCAA (Seq. Id. 13) | 32279rev | GGGAATGTTGATGTCATTCAGAAA (Seq. Id. 14) |
| 32757_at | 32757for | GCCTGTTGCAGAGTTTTTCTGTAA (Seq. Id. 15) | 32757rev | TTCCAATATTCTCTAACACGTACAC TGAA (Seq. Id. 16) |
| 32814_at | 32814for | GGTAGAAGAAACAATGCAAGACATACA T (Seq. Id. 17) | 32814rev | CCCTTGTTAATGATGCCTGTTCTAT (Seq. Id. 18) |
| 33372_at | 33372for | GGAAATGTACCTGAAAAGGATTTTAGA (Seq. Id. 19) | 33372rev | ACTACATCCCCTCCATGTGCAT (Seq. Id. 20) |
| 33890_at | 33890for | AGTGCAGATTTATACTCCTGACGTGT (Seq. Id. 21) | 33890rev | CAAGACTTATAATCATGAAATACAG AATTAAAAGTT (Seq. Id. 22) |
| 34250_at | 34250for | TGTCCGTCCCTGTTTTTGCT (Seq. Id. 23) | 34250rev | CACCCTGCCTTTCCCTAGAGA (Seq. Id. 24) |
| 34375_AT | 34375for | GGAAGATCTCAGTGCAGAGGCT (Seq. Id. 25) | 34375rev | GATCTCCTTGGCCACAATGGT (Seq. Id. 26) |
| 34582_at | 34582for | TGGATGGAGGACAGATTGTGACT (Seq. Id. 27) | 34582rev | AATGAGGAGCATGGTGACCAG (Seq. Id. 28) |
| 34777_AT | 34777for | TCGCCCACAAACTGATTTCTC (Seq. Id. 29) | 34777rev | ACGCATTGCACTTTTCCTCTTT (Seq. Id. 30) |
| 35109_at | 35109for | CCCTCCTGCTGTGCTCTTCT (Seq. Id. 31) | 35109rev | GTGACAGACACACATCAGCCACTA (Seq. Id. 32) |
| 35680_r_a t | 35680_rfor | GATAGAATTAACTCGTATTTTTCTATGG TTTTAA (Seq. Id. 33) | 35680_rre v | GGAGAAAGCCAAGGGAAACAA (Seq. Id. 34) |
| 35857_at | 35857for | TCCTTACTTGTCATCAGAGACGAACT (Seq. Id. 35) | 35857rev | AAAAATCTGTAGGGAATGCATCCTT (Seq. Id. 36) |
| 36254_AT | 36254for | AAAGGAAGGAAACTCCTGACAGTCT (Seq. Id. 37) | 36254rev | GCTTCAGAGATACAAATACAGTGTA ATACCA (Seq. Id. 38) |
| 36672_at | 36672for | CTGGAGTAGAGTTCCTGGTTGCTT (Seq. Id. 39) | 36672rev | AAAGACACTTCAACCTCAAAACCAA (Seq. Id. 40) |
| 36694_at | 36694for | CCTCAGTGGGTTCGTTAAAATCA (Seq. Id. 41) | 36694rev | GGCATTTAATTGACCTCACAGAGA (Seq. Id. 42) |
| 36711_at | 36711 for | GTGCCAATATGCCCTCCAAA (Seq. Id. 43) | 36711 rev | GGCATCTCTTCAGTGCAATTTCT (Seq. Id. 44) |
| 37015_AT | 37015for | TTCTGAAATGTGACCCCCAAGT (Seq. Id. 45) | 37015rev | GCTAAATGCAACTGTTCCTTTTCTA TAA (Seq. Id. 46) |
| 37183_at | 37183 for | GGCAACATTTCCGAAGACCTT (Seq. Id. 47) | 37183rev | GCATACTCTAAAACGCACAGTGTGA (Seq. Id. 48) |
| 37230_AT | 37230 for | CCTCCCTCCAGTGTCCACAT (Seq. Id. 49) | 37230rev | AAGCAGGATTCTGGACATGGAA (Seq. Id. 50) |
| 38111_at | 38111 for | AAGTGCTAATAATTAACTCAACCAGGT CTA (Seq. Id. 51) | 38111 rev | CTAGGTCTATATCCGTATGAAATGC ATT (Seq. Id. 52) |
| 38112_g_ at | 38112_g for | AAGTCACAATGAGTTTGGGCATATT (Seq. Id. 53) | 38112_gr ev | ACCATTTCCAGCCTAAACTACATAA AA (Seq. Id. 54) |
| 38404_at | 38404 for | CAGCTTTGACTTTATCTCCTGCTCTT (Seq. Id. 55) | 38404rev | CCCTGGAGGCTGTGATCTCA (Seq. Id. 56) |
| 39114_at | 39114for | GAGTCTGAAGGACCCTAGTTCCTAGA (Seq. Id. 57) | 39114rev | TCTGTCCCTTCACCTCTGATCA (Seq. Id. 58) |
| 39397_at | 39397for | AATTGTTTTTCGTCCGTTTGGTA (Seq. Id. 59) | 39397rev | AATTGCCATATACGGCCAGTTAA (Seq. Id. 60) |
| 39579_at | 39579for | GCAGGAGCCTCACTGTGCAT (Seq. Id. 61) | 39579rev | ACAGATGTGGCCCCGTTGTA (Seq. Id. 62) |
| 39599_at | 39599p2F | TGTACACATATGAACGCACAACATG (Seq. Id. 63) | 39599p2R | GACCTCAGGAAAAGGCTTCAGA (Seq. Id. 64) |
| 39839_at | 39839p2F | GTCCAAACCAGCCGTCTGTT (Seq. Id. 65) | 39839p2R | GCCTCTTTGCCATCTTGTGAA (Seq. Id. 66) |
| 40225_AT | 40225for | AAAGCCTCTGATTGTTGTTTCCTT (Seq. Id. 67) | 40225rev | ACGCCCGAAACACCAAATAA (Seq. Id. 68) |
| 40382_at | 40382for | AGCAACAGGAAACTCATGGAGATT (Seq. Id. 69) | 40382rev | GAGCAGCGCTATGGTACAGAATAC T (Seq. Id. 70) |
| 40425_at | 40425for | CAGCCTCAAAACGGGTCAGT (Seq. Id. 71) | 40425rev | CTTCTTTCTCCGTCCCTCCG (Seq. Id. 72) |
| 40646_AT | 40646for | AATATCCCCTCCCAGTCACCTT (Seq. Id. 73) | 40646rev | AAAGCTGCTCCCATAGCCCT (Seq. Id. 74) |
| 41016_at | 41016for | TGACACCTGTGCATTAGATGCA (Seq. Id. 75) | 41016rev | CATGGTGTGAGTCTGGGCCT (Seq. Id. 76) |
| 41439_at | 41439for | TGGTACAGGGTGCCTATTTTAGTCA (Seq. Id. 77) | 41439rev | TGTAGTTTCCAAAATGAACCCTTGT (Seq. Id. 78) |
| 41531_AT | 41531for | AACTGCCTTGTGTTCTGTGAGAAA (Seq. Id. 79) | 41531rev | GCAGCATTGTAAGTTGTGATGCA (Seq. Id. 80) |
| 875_G_AT | 875_Gfor | TTGAACACTCACTCCACAACCC (Seq. Id. 81) | 875_Grev | TTTCACATCAACAAACAAAATTCATT ATAA (Seq. Id. 82) |

RNA levels are then measured using Q-PCR. Briefly, cDNA is synthesized using random hexamers, diluted in a master mix containing TAQ polymerase, SybrGreen^{™} (Molecular Probes, Inc., Eugene, Oregon), unlabeled nucleotides, buffer and water. The mixture is aliquotted into TaqMan® plates (Perkin Elmer) and pairs of oligonucleotides are added to the appropriate wells. Each sample is assayed in at least duplicate wells and every sample is assayed with every oligonucleotide pair where the transcriptase is omitted from the first reaction (noRT controls). The threshold cycle (CT) is calculated using Perkin Elmer software ABI Prism ® 7700 Sequence Detection System Revison B. The CT value is defined as the cycle at which a statistically significant increase in fluorescence (from the SybrGreen^{™}) is detected. A lower CT value is indicative of a higher mRNA concentration.

cDNA is separately prepared from all the N1 and S1 samples according to conventional methods. Yield is estimated using PicoGreen^{™} (Molecular Probes, Inc., Eugene, Oregon) assays. Equal amounts of cDNA from all N1 samples are mixed into one pooled sample, and equal amounts of cDNA from all the S1 samples are mixed into a separate pooled sample. Q-PCR with oligonucleotides designed for GAPDH and the 38 genes identified from the GeneChip® experiments are executed in duplicate with no RT controls. Genes with a greater than 1 cycle difference in C_{T} between the two groups are chosen for confirmation by Q-PCR on individual samples.

The 9 genes with the largest differences in CT between N1 and S1 pooled samples are identified and Q-PCR is run on the individual cDNA samples. The individual CT values for these 9 genes plus the GAPDH (as control) are examined and t-test p-values are calculated to test the null hypothesis that the two samples N1 and S1 are derived from the same population. Five genes are found to be differentially expressed between the normal and schizophrenic anterior cingulate samples. These genes have the designation 34777_at, 36711_at, 39114_at, 39839_at and 39579_at.

To confirm the differential resolution of these five genes, an independent set of anterior cingulate samples is obtained (Maryland Psychiatric Research Clinic). Nine normal samples and 9 schizophrenic samples are designated N2 and S2, respectively. For each of the 5 genes, plus GAPDH, standard curves are obtained by Q-PCR of a 2 fold dilution series of pooled normal cDNA with all 10 pairs of primers. Plotting C_{T} against the logarithm of the starting amount of cDNA in microliters is linear. The equations of these standard curves can be used to calculate the relative amounts of cDNA to a gene, GAPDH, that is regarded as unchanging between the samples. RNA samples are obtained from these new samples and both the old and new samples are subjected to Q-PCR. From the C_{T}'s the relative amounts of cDNA corrected for the amount of GAPDH in each sample are calculated for all the genes and samples

The analysis of variance in the data demonstrated that three of these genes, 39114_at, 39839_at and 34777_at have consistently different expression levels in the normal and schizophrenic anterior cingulate samples over both data sets with p-values of 0.001, 0.0013 and 0.0013, respectively.

BLAST (Basic Local Alignment Search Tool) searches of the public sequence databases identified 39114_at, 34777_at, and 39839_at as decidual protein induced by progesterone (DEPP), adrenomedullin, and cold shock domain protein A (csdA) respectively.

## Claims

1. A method of screening for schizophrenia in a population comprising determining, in vitro, the magnitude of expression, in members of the population, of at least one gene selected from the group consisting of the gene encoding decidual protein induced by progesterone (DEPP), the gene encoding adrenomedullin and the gene encoding cold shock domain protein A (csdA) in a sample and comparing the magnitude of expression to a baseline magnitude of expression of the gene, wherein increased gene expression indicates the presence of schizophrenia.

2. A method for diagnosing schizophrenia in a host comprising determining, in vitro, the magnitude of expression of at least one gene selected from the group consisting of the gene encoding decidual protein induced by progesterone (DEPP), the gene encoding adrenomedullin and the gene encoding cold shock domain protein A (csdA) in a sample and comparing the magnitude of expression to a baseline magnitude of expression of the gene, wherein increased gene expression indicates the presence of schizophrenia.

3. A method according to claim 1 or 2 wherein the sample is taken from brain, spinal cord, lymphatic fluid, blood, urine or feces.

4. A method according to claim 3 wherein the sample is taken from the anterior cingulate.

5. A method according to claim 1, 2, 3 or 4 wherein the sample is from a human.

6. Use of a transgenic nonhuman animal expressing a transgene of at least one of the genes selected from the group consisting of the gene encoding decidual protein induced by progesterone (DEPP), the gene encoding adrenomedullin and the gene encoding cold shock domain protein A (csdA) wherein the resulting expression of the at least one gene is higher than baseline, for determining the effects of therapeutic or potentially therapeutic compounds on schizophrenia.

7. The use of Claim 6 wherein expression of said gene is enhanced in the transgenic nonhuman animal by one or more alterations in regulatory sequences of the gene such that the gene is expressed at higher than baseline levels.

8. The use of Claim 6 wherein expression of said gene is enhanced in the transgenic nonhuman animal by an increased copy number of said gene.

9. The use according to claim 6, 7 or 8 wherein the transgenic nonhuman animal is a mammal.

10. The use according to claim 7, 8 or 9 wherein the one or more alterations comprises substitution of a promoter having a higher rate of expression than the native promoter of the gene.

11. The use according to claim 10 wherein the promoter is an inducible promoter.

12. A method of screening for a compound useful in the treatment of schizophrenia comprising operatively linking a reporter gene which expresses a detectable protein to a regulatory sequence for a gene selected from the group consisting of DEPP, adrenomedullin and csdA to produce a reporter construct; transfecting a cell with the reporter construct; exposing the transfected cell to a test compound; and comparing the level of expression of the reporter gene after exposure to the test compound to the level of expression before exposure to the test compound, wherein a lower level of expression after exposure is indicative of a compound useful for the treatment of schizophrenia.

## Patentansprüche

1. Verfahren zum Screenen auf Schizophrenie in einer Population, das umfasst die in vitro Bestimmung des Expressionsausmaßes zumindest eines Gens bei Vertretern einer Population, das ausgewählt ist aus der Gruppe, die besteht aus dem Gen, das für das durch Progesteron induzierte Decidualprotein (DEPP) kodiert, dem Gen, das für Adrenomedullin kodiert und dem Gen, das für das Kälteschockdomänenprotein A (CsdA) in einer Probe kodiert und den Vergleich der Größenordnung der Expression mit einer Grundliniengrößenordnung der Expression des Gens umfasst, worin die erhöhte Genexpression das Vorkommen von Schizophrenie anzeigt.

2. Verfahren zur Diagnose von Schizophrenie bei einem Wirt, das die in vitro Bestimmung der Expressionsgrößenordnung von mindestens einem Gen, das ausgewählt ist aus der Gruppe, die besteht aus dem Gen, das für das durch Progesteron induzierte Decidualprotein (DEPP) kodiert, dem Gen, das für Adrenomedullin kodiert und dem Gen, das für das Kälteschockdomänenprotein A (CsdA) kodiert in einer Probe und den Vergleich der Größenordnung der Expression mit einer Grundliniengrößenordnung der Expression des Gens umfasst, worin die erhöhte Genexpression das Vorkommen von Schizophrenie anzeigt.

3. Verfahren nach Anspruch 1 oder 2, worin die Probe aus dem Gehirn, dem Spinalstrang, der lymphatischen Flüssigkeit, dem Blut, dem Urin oder dem Stuhl entnommen wird.

4. Verfahren nach Anspruch 3, worin die Probe aus dem anterioren Cingluate entnommen wird.

5. Verfahren nach Anspruch 1, 2, 3 oder 4, worin die Probe von einem Menschen stammt.

6. Verwendung eines transgenen Tieres, das ein Transgen aus zumindest einem der Gene exprimiert, das ausgewählt ist aus der Gruppe, die besteht aus dem Gen, das für das durch Progesteron induzierte Decidualprotein (DEPP) kodiert, dem Gen, das für Adrenomedullin kodiert und dem Gen, das für das Kälteschockdomänenprotein A (CsdA) kodiert, worin die entstehende Expression des zumindest einen Gens höher als die Grundlinie ist, um die Effekte von therapeutischen oder potentiell therapeutischen Verbindungen für Schizophrenie zu bestimmen.

7. Verwendung nach Anspruch 6, worin die Expression des Gens im transgenen Tier durch eine oder mehrere Veränderungen der regulatorischen Sequenzen des Gens so erhöht ist, dass das Gen stärker als die Grundlinienspiegel exprimiert wird.

8. Verwendung nach Anspruch 6, worin die Expression des Gens im transgenen Tier durch eine erhöhte Kopiezahl des Gens erhöht ist.

9. Verwendung nach Anspruch 6, 7 oder 8, worin das transgene Tier ein Säuger ist.

10. Verwendung nach Anspruch 7, 8 oder 9, worin die eine oder mehrere Veränderungen eine Substitution eines Promotors umfassen, der eine höhere Expressionsrate als der native Promotor des Gens aufweist.

11. Verwendung nach Anspruch 10, worin der Promotor ein induzierbarer Promotor ist.

12. Verfahren zum Screenen einer Verbindung, die zur Behandlung der Schizophrenie brauchbar ist, das umfasst die operative Anbindung eines Reportergens, das ein detektierbares Protein exprimiert, an eine regulatorische Sequenz für ein Gen, das aus der Gruppe ausgewählt ist, die besteht aus DEPP, Adrenomedullin und CsdA, zur Herstellung eines Reporterkonstrukts, Transfektion einer Zelle mit dem Reporterkonstrukt, Aussetzen der transfizierten Zelle gegenüber einer Testverbindung und Vergleich der Expressionsmenge des Reportergens nach der Exposition gegenüber der Testverbindung mit der Expressionsmenge vor der Exposition gegenüber der Testverbindung, worin eine geringere Expressionsmenge nach der Exposition eine Verbindung anzeigt, die zur Behandlung der Schizophrenie brauchbar ist.

## Revendications

1. Méthode de dépistage de la schizophrénie chez une population comprenant les étapes consistant à déterminer, *in vitro,* l'amplitude de l'expression, chez les membres de cette population, d'au moins un gène choisi parmi le groupe comportant le gène codant la protéine déciduale induite par la progestérone (DEPP), le gène codant l'adrénomédulline et le gène codant le domaine de « Choc Froid » de la protéine A (csdA) dans un échantillon, et à comparer cette amplitude d'expression par rapport à une amplitude de base d'expression du gène, où l'augmentation de l'expression du gène est indicatrice de la présence d'une schizophrénie.

2. Méthode de diagnostic de la schizophrénie chez un hôte comprenant les étapes consistant à déterminer, *in vitro,* l'amplitude de l'expression d'au moins un gène choisi parmi le groupe comportant le gène codant la protéine déciduale induite par la progestérone (DEPP), le gène codant l'adrénomédulline et le gène codant le domaine de « Choc Froid » de la protéine A (csdA) dans un échantillon et à comparer cette amplitude d'expression par rapport à une amplitude de base d'expression du gène, où l'augmentation de l'expression du gène est indicatrice de la présence d'une schizophrénie.

3. Procédé selon la revendication 1 ou 2, dans lequel l'échantillon est extrait du cerveau, de la moelle épinière, du liquide lymphatique, du sang, de l'urine ou des fèces.

4. Procédé selon la revendication 3, dans lequel l'échantillon est extrait du cingulum antérieur.

5. Procédé selon la revendication 1, 2, 3 ou 4, dans lequel l'échantillon est prélevé chez l'homme.

6. Utilisation d'un animal transgénique non humain exprimant un transgène d'au moins l'un des gènes choisis parmi le groupe comportant le gène codant la protéine déciduale induite par la progestérone (DEPP), le gène codant l'adrénomédulline et le gène codant le domaine de « Choc Froid » de la protéine A (csdA) où l'expression résultant du au moins un gène est supérieure à l'expression de base, pour déterminer les effets de composés thérapeutiques ou potentiellement thérapeutiques sur la schizophrénie.

7. Utilisation selon la revendication 6, dans laquelle l'expression dudit gène est stimulée dans l'animal transgénique non humain par une ou plusieurs altérations des séquences régulatrices du gène de telle sorte que le gène est exprimé à des taux supérieurs aux taux de base.

8. Utilisation selon la revendication 6, dans laquelle l'expression dudit gène est stimulée dans l'animal transgénique non humain par une augmentation du nombre de copies dudit gène.

9. Utilisation selon la revendication 6, 7 ou 8, dans laquelle l'animal transgénique non humain est un mammifère.

10. Utilisation selon la revendication 7, 8 ou 9, dans laquelle l'altération ou les altérations comprend/comprennent la substitution d'un promoteur ayant un taux d'expression supérieur au promoteur natif du gène.

11. Utilisation selon la revendication 10, dans laquelle le promoteur est un promoteur inductible.

12. Procédé de criblage d'un composé utile dans le traitement de la schizophrénie comprenant les étapes consistant à lier, de façon fonctionnelle, un gène reporter qui exprime une protéine détectable à une séquence régulatrice d'un gène choisi parmi le groupe comprenant la DEPP, l'adrénomédulline et la csdA pour produire une construction reporter ; à transfecter une cellule avec la construction reporter ; à exposer la cellule transfectée à un composé test ; et à comparer le taux d'expression du gène reporter après exposition au composé test avec le taux d'expression avant exposition au composé test, où un taux d'expression inférieur après exposition est indicateur d'un composé utile pour traiter la schizophrénie.
